# EUROPÄISCHE PATENTSCHRIFT

(11) **EP 4 076 226 B1**
(45) Veröffentlichungstag und Bekanntmachung des Hinweises auf die Patenterteilung: **29.10.2025**
(21) Anmeldenummer: 20839273.8
(22) Anmeldetag: 17.12.2020
(51) Int. Cl.: A61B 17/29, A61B 17/295, A61B 18/14

(54) **MEDIZINISCHES INSTRUMENT**
MEDICAL INSTRUMENT
INSTRUMENT MÉDICAL

(30) Priorität: 20.12.2019 DE 102019135513
(43) Veröffentlichungstag der Anmeldung: 26.10.2022
(73) Patentinhaber: Aesculap AG, 78532 Tuttlingen (DE)
(72) Erfinder: WALBERG, Erik, 78532 Tuttlingen (DE); HEIZMANN, Patrick, 78183 Hüfingen (DE)
(74) Vertreter: Hoeger, Stellrecht & Partner Patentanwälte mbB
(86) Internationale Anmeldenummer: PCT/EP2020/086698
(87) Internationale Veröffentlichungsnummer: WO 2021/122940

(56) Entgegenhaltungen:
- EP-A1- 3 305 223
- US-A1- 2008 015 566
- US-A1- 2017 056 038

## Beschreibung

Die vorliegende Offenbarung betrifft ein medizinisches Instrument mit einem distalen und einem proximalen Ende, wobei am distalen Ende ein erstes Werkzeugelement und ein zweites Werkzeugelement relativ zueinander bewegbar angeordnet oder ausgebildet sind, wobei die Werkzeugelemente eine Werkzeugelementerstreckungsrichtung von proximal nach distal definieren, wobei das erste Werkzeugelement eine erste Werkzeugelementfläche und das zweite Werkzeugelement eine zweite Werkzeugelementfläche definieren, wobei die erste Werkzeugelementfläche und die zweite Werkzeugelementfläche aufeinander zu weisen, wobei die Werkzeugelemente im Bereich der Werkzeugelementflächen eine Breite definieren, welche sich in einer Richtung quer, insbesondere senkrecht, zur Werkzeugelementerstreckungsrichtung und quer, insbesondere senkrecht, zu einer Flächennormalen der ersten Werkzeugelementfläche und/oder der zweiten Werkzeugelementfläche erstreckt, wobei das erste Werkzeugelement und/oder das zweite Werkzeugelement mindestens ein Greifelement mit einer vom jeweiligen Werkzeugelement weg weisenden Greiffläche aufweist, wobei die Breite des Werkzeugelements ausgehend vom proximalen Ende des mindestens einen Werkzeugelements zum distalen Ende desselben hin abnimmt, wobei die Breite mindestens ein lokales Minimum zwischen dem proximalen und dem distalen Ende aufweist und wobei die Greiffläche im Bereich des lokalen Minimums der Breite ausgebildet ist, wobei das erste Werkzeugelement und/oder das zweite Werkzeugelement einen plattenförmigen Werkzeugelementbereich umfassen und wobei die Werkzeugelementfläche am plattenförmigen Werkzeugelementbereich des jeweiligen Werkzeugelements ausgebildet ist.

Es ist bekannt, medizinische Instrumente mit zwei relativ zueinander bewegbaren, insbesondere verschieb- und/oder verschwenkbaren, Werkzeugelementen zur Ausbildung eines Instrumentenmauls auszustatten, wobei bei diesen Instrumenten die Werkzeugelementerstreckungsrichtung nicht geradlinig, sondern gekrümmt verläuft. Derart gekrümmte distale Enden medizinischer Instrumente, die durch die auch als Maulteile bezeichneten, gekrümmten Werkzeugelemente ausgebildet werden, ermöglichen dem Anwender insbesondere eine verbesserte Sichtbarkeit des distalen Endes des Instruments sowie verbesserte Präparationseigenschaften. Beispielsweise kann das Instrument aufgrund seiner gekrümmten Maulteile auch als eine Art seitlicher Haken genutzt werden, um Gewebe und Gefäße besser zu greifen und diese dann beispielsweise zwischen den Werkzeugelementflächen der Werkzeugelemente zu positionieren.

Derart vorteilhafte Eigenschaften weisen Instrumente mit Werkzeugelementen, die eine geradlinige Werkzeugelementerstreckungsrichtung definieren, nicht auf.

In der EP 3 305 223 A1 ist eine chirurgische Operationsvorrichtung beschrieben. Aus der US 2017/0056038 A1 ist ein chirurgisches Maulteil für Präparationszwecke bekannt. Die US 2008/0015566 A1 offenbart ein chirurgisches Versiegelungs- und Schneidgerät.

Es ist daher eine Aufgabe der vorliegenden Erfindung, ein medizinisches Instrument der eingangs beschriebenen Art so zu verbessern, dass eine gute Handhabung von Gewebe mit diesem Instrument, insbesondere auch bei einem geradlinigen Verlauf der Werkzeugelementerstreckungsrichtung, ermöglicht wird.

Diese Aufgabe wird bei einem medizinischen Instrument der eingangs beschriebenen Art erfindungsgemäß dadurch gelöst, dass das distale Ende des jeweiligen Werkzeugelements durch den plattenförmigen Werkzeugelementbereich gebildet ist.

Die vorgeschlagene Weiterbildung bekannter medizinischer Instrumente hat insbesondere den Vorteil, dass sich die vorteilhaften Eigenschaften gekrümmter Maulteile auch bei geradlinig verlaufenden Maulteilen realisieren lassen. Zudem ist es insbesondere möglich, geradlinig verlaufende Maulteile beidseitig mit Greifelementen, die entsprechende Greifflächen definieren, auszubilden. Insbesondere ist es möglich, nur am ersten Werkzeugelement oder nur am zweiten Werkzeugelement ein oder zwei oder noch mehr Greifelemente vorzusehen. Es können aber auch an beiden Werkzeugelementen jeweils eine, zwei oder mehr, beispielsweise drei oder vier, Greifelemente angeordnet oder ausgebildet sein. So kann auch ein solches Instrument für feine Präparationen eingesetzt werden. Mit anderen Worten lässt sich so bei herkömmlichen Instrumenten eine feine Präparationseigenschaft erreichen. Zudem wird auch die Sichtbarkeit einer distalen Instrumentenspitze verbessert, da insbesondere das mindestens eine lokale Minimum der Breite am mindestens einen Werkzeugelement gut sichtbar ist. Gemäß der Erfindung ist vorgesehen, dass das erste Werkzeugelement und/oder das zweite Werkzeugelement einen plattenförmigen Werkzeugelementbereich umfassen und dass die Werkzeugelementfläche am plattenförmigen Werkzeugelementbereich des jeweiligen Werkzeugelements ausgebildet ist. Ein solcher plattenförmiger Werkzeugelementbereich weist insbesondere eine Dicke in Richtung der Flächennormalen der jeweiligen Werkzeugelementfläche auf. Diese Dicke ist im Vergleich zur Breite einerseits und zur Erstreckung des Werkzeugelements in der Werkzeugelementerstreckungsrichtung anderseits deutlich kleiner, insbesondere kleiner als 50%, und ermöglicht so insbesondere die Ausbildung einer insbesondere zur Präparation von Gewebe geeigneten Kontur, welche schieberartig und insbesondere stumpf ausgebildet werden kann, um Gewebeschichten voneinander zu lösen. Eine Dicke des Werkzeugelements insgesamt kann insbesondere deutlich größer sein als eine Dicke des plattenförmigen Werkzeugelementbereichs. Insbesondere kann der plattenförmige Werkzeugelementbereich in Form eines mindestens teilweise einen Rand des Werkzeugelements bildenden flanschartigen Bereichs ausgebildet sein, mit dem in distaler Richtung und beidseitig zur Werkzeugelementerstreckungsrichtung Gewebe präpariert werden kann. Einen plattenförmigen Werkzeugelementbereich an einem der zwei Werkzeugelemente oder an beiden Werkzeugelementen auszubilden hat beispielsweise den Vorteil, dass eine Bewegung der beiden Werkzeugelemente relativ zueinander insbesondere eine stumpfe, also atraumatische Gewebepräparation durch Spreizen von Gewebe ermöglicht. Gemäß der Erfindung ist vorgesehen, dass das distale Ende des jeweiligen Werkzeugelements durch den plattenförmigen Werkzeugelementbereich gebildet ist. So kann die Spitze auf einfache Weise und ohne großen Kraftaufwand zwischen Gewebeschichten eingeführt werden, um diese voneinander zu trennen.

Die Handhabbarkeit von Gewebe und Gefäßen mit dem medizinischen Instrument lässt sich insbesondere dadurch weiter verbessern, dass das erste Werkzeugelement und/oder das zweite Werkzeugelement zwei Greifelemente umfassen. Dadurch hat ein Operateur mehrere Optionen, Gewebe zu präparieren und beispielsweise mit den zwei Greifelementen des einen und/oder des anderen Werkzeugelements zurückzuhalten.

Eine weitere Verbesserung der Handhabung des medizinischen Instruments zum Präparieren von Gewebe und Gefäßen kann insbesondere dadurch erreicht werden, dass die zwei Greifelemente jeweils eine Greiffläche definieren, die im Bereich des lokalen Minimums der Breite und voneinander weg weisend angeordnet oder ausgebildet sind. So kann beispielsweise bei geradlinig verlaufenden Werkzeugelementen oder Maulteilen beidseitig ein Greifelement mit jeweils einer Greiffläche angeordnet oder ausgebildet werden. So kann mit den Werkzeugelementen beidseitig eine Hakenfunktion realisiert werden. Bei gekrümmten Maulteilen ist dies nur einseitig möglich. Bei geradlinig verlaufenden Werkzeugelementen, die nicht die Eigenschaft haben, dass die Breite ein lokales Minimum aufweist, ist dies ebenfalls nicht möglich. Gewebe rutscht bei derartigen Werkzeugelementen stets in distaler Richtung an beziehungsweise von den Werkzeugelementen ab. Es ist mit solchen Instrumenten nicht möglich, Gewebe oder Gefäße in definierter Weise zurückzuhalten und ein Abrutschen derselben zu verhindern oder zumindest zu erschweren.

Vorteilhaft ist es, wenn die Greiffläche von der Werkzeugelementerstreckungsrichtung weg weist. Mit anderen Worten kann die Greiffläche auf diese Weise seitlich vom jeweiligen Werkzeugelement weg weisen.

Günstig ist es, wenn das erste Werkzeugelement und das zweite Werkzeugelement in Form von Klemmelementen ausgebildet sind und wenn die erste Werkzeugelementfläche und die zweite Werkzeugelementfläche in Form von Klemmflächen ausgebildet sind. Ein derart ausgestattetes medizinisches Instrument kann beispielsweise als Fasszange eingesetzt werden oder als bipolares Versiegelungsinstrument.

Eine Hakenfunktion kann mit den Werkzeugelementen des Instruments auf einfache Weise beispielsweise dadurch realisiert werden, dass die Greiffläche des mindestens einen Werkzeugelements vom jeweiligen Werkzeugelement weg weisend konkav gekrümmt ist. Eine solche Ausgestaltung ermöglicht es insbesondere, eine in distaler Richtung divergierende Form der Werkzeugelemente zu realisieren, also auch bei einer geradlinigen Erstreckung der Werkzeugelemente eine hakenartige Form, mit der sich Gewebe und Gefäße einfach und in gewünschter Weise zurückhalten und präparieren lassen.

Das Instrument lässt sich insbesondere auf einfache und kompakte Weise ausbilden, wenn das mindestens eine Greifelement einen Teilbereich einer Seitenfläche des jeweiligen Werkzeugelements umfasst. So kann das Greifelement insbesondere einstückig mit dem Werkzeugelement ausgebildet und in dieses integriert werden.

Vorteilhaft ist es, wenn der plattenförmige Werkzeugelementbereich die Seitenfläche des jeweiligen Werkzeugelements definiert. So kann insbesondere eine sehr schmale, hakenförmige Greiffläche durch die Seitenfläche des dünnen plattenförmigen Werkzeugelementbereichs ausgebildet werden. Gewebe kann so besonders einfach und sicher zurückgehalten werden.

Um insbesondere Stabilität der Werkzeugelemente zu verbessern, ist es günstig, wenn der plattenförmige Werkzeugelementbereich auf einer von der jeweiligen Werkzeugelementfläche weg weisenden Seite eine Stützstruktur trägt. Die Stützstruktur kann insbesondere eine Erstreckung in Richtung der Dicke des plattenförmigen Werkzeugelementbereichs aufweisen, der die Dicke um ein Mehrfaches ihrer Erstreckung übersteigt.

Um insbesondere einen distalen Endbereich des Werkzeugelements durch den plattenförmigen Werkzeugelementbereich ausbilden zu können, ist es vorteilhaft, wenn die Stützstruktur bezogen auf das distale Ende des Werkzeugelements in proximaler Richtung zurückgesetzt ist. Ferner kann sie zudem optional auch seitlich bezogen auf die Seitenflächen des plattenförmigen Werkzeugelementbereichs zurückgesetzt sein. So kann in distaler Richtung und optional auch auf einer oder beiden Seiten der Werkzeugelemente ein Präparationselement, insbesondere ein stumpfes, ausgebildet werden, um Gewebe zu trennen und zu präparieren.

Um die Verletzung von Gewebe zu vermeiden, ist es vorteilhaft, wenn die Greiffläche des mindestens einen Greifelements tangentenstetig gekrümmt ist.

Vorteilhaft ist es, wenn die Breite mindestens ein lokales Maximum zwischen dem proximalen und dem distalen Ende aufweist und wenn sich die Greiffläche in distaler Richtung bis zu dem lokalen Maximum erstreckt. So kann insbesondere ein- und/oder beidseitig am jeweiligen Werkzeugelement eine hakenförmige Greiffläche ausgebildet werden, mit der sich Gewebe und Gefäße einfach zurückhalten lassen.

Vorteilhafterweise nimmt die Breite in distaler Richtung ausgehend vom lokalen Maximum kontinuierlich ab. Insbesondere kann sie bis zum distalen Ende hin kontinuierlich abnehmen. In diesem Fall ist dann das Werkzeugelement im Bereich des lokalen Maximums breiter, beispielsweise mehr als 50% breiter, als im Bereich des in proximaler Richtung versetzten lokalen Minimums der Breite.

Günstig ist es, wenn ein Abstand des lokalen Minimums der Breite von einem proximalen Ende der Greiffläche größer ist als von einem distalen Ende der Greiffläche. Diese Ausgestaltung ermöglicht es insbesondere, die Hakenfunktion der Werkzeugelemente möglichst weit in distaler Richtung des Instruments auszubilden. So kann Gewebe insbesondere mit einer Spitze des Instruments oder einem Bereich nahe der Spitze des Instruments wie beschrieben präpariert werden.

Ferner kann es vorteilhaft sein, wenn die zwei Greifelemente symmetrisch am jeweiligen Werkzeugelement angeordnet oder ausgebildet sind. So kann insbesondere eine Handhabung des Instruments für den Operateur weiter verbessert werden. Unabhängig von einer Orientierung der Werkzeugelemente bezogen auf die Werkzeugelementerstreckungsrichtung kann er so frei und nahezu uneingeschränkt in zwei voneinander entgegengesetzten Richtungen Gewebe präparieren und halten.

Das Instrument lässt sich auf einfache und kostengünstige Weise ausbilden, wenn das erste und/oder das zweite Werkzeugelement spiegelsymmetrisch bezogen auf eine die Werkzeugelementerstreckungsrichtung definierende Längsachse des jeweiligen Werkzeugelements enthaltende Spiegelebene ausgebildet sind.

Um Gewebe bei einem chirurgischen Eingriff im menschlichen oder tierischen Körper sicher und gefühlvoll präparieren und halten zu können, ist es günstig, wenn eine Krümmung der Greiffläche des mindestens einen Greifelements einen Krümmungsradius in einem Bereich von etwa 5 mm bis etwa 30 mm definiert. Insbesondere kann der Krümmungsradius etwa 15 mm betragen. Derart gekrümmte Greifflächen eignen sich hervorragend für die Präparation und zum Zurückhalten von Gewebe.

Gemäß einer weiteren bevorzugten Ausführungsform der Erfindung kann vorgesehen sein, dass die Greiffläche des mindestens einen Greifelements am ersten Werkzeugelements und die Greiffläche des mindestens einen Greifelements am zweiten Werkzeugelement in der Werkzeugelementerstreckungsrichtung überlappend oder in distaler oder proximaler Richtung zueinander versetzt angeordnet oder ausgebildet sind. So können die Werkzeugelemente mit ihren Greifelementen, die die Greifflächen definieren, in unterschiedlicher Weise und damit auch für unterschiedliche Präparationszwecke optimiert ausgebildet werden. Insbesondere eine versetzte Anordnung ermöglicht es, beispielsweise bei voneinander weg bewegten Werkzeugelementen, Gewebe in unterschiedlichen Abständen zueinander in der Werkzeugelementerstreckungsrichtung zu halten.

Vorzugsweise ist das mindestens eine Greifelement einstückig am jeweiligen Werkzeugelement angeformt oder ausgebildet. Dies ermöglicht eine besonders einfache Herstellung des Instruments, insbesondere der Werkzeugelemente.

Günstig kann es jedoch auch sein, wenn das mindestens eine Greifelement mit dem jeweiligen Werkzeugelement lösbar verbindbar ausgebildet ist. So können beispielsweise Greifelemente bei Bedarf ausgetauscht werden, um Instrumente bei unterschiedlichen chirurgischen Eingriffen optimiert einsetzen zu können. Ferner können auch bei entsprechender Ausgestaltung der Werkzeugelemente Greifelemente an diesen nachgerüstet werden. Werden Greifelemente beispielsweise nicht benötigt, können sie von den Werkzeugelementen entfernt werden.

Auf einfache Weise lässt sich das Instrument ausbilden, wenn das mindestens eine Greifelement und das zugeordnete Werkzeugelement in einer Verbindungsstellung kraft- und/oder formschlüssig in Eingriff stehen. Insbesondere lässt sich so eine lösbare Verbindbarkeit zwischen Greifelement und Werkzeugelement auf einfache Weise realisieren.

Günstig ist es, wenn das das mindestens eine Greifelement näher am distalen Ende als am proximalen Ende des jeweiligen Werkzeugelements angeordnet oder ausgebildet ist. So kann beispielsweise eine Rückhalte- oder Hakenfunktion des Greifelements weiter distal am Instrument realisiert werden.

Vorteilhaft ist es, wenn die Greiffläche des mindestens einen Greifelements quer, insbesondere senkrecht, zur Werkzeugelementfläche des jeweiligen Werkzeugelements verläuft. Damit lässt sich eine Haken- oder Rückhaltefunktion in einer Richtung realisieren, welche quer zu einer Werkzeugelementerstreckungsrichtung, die die Werkzeugelemente vorgeben, verläuft.

Gemäß einer weiteren bevorzugten Ausführungsform der Erfindung kann vorgesehen sein, dass das mindestens eine Greifelement eine weitere Greiffläche aufweist und dass die weitere Greiffläche bezogen auf die Werkzeugelementfläche des jeweiligen Werkzeugelements geneigt ist. Durch diese Ausgestaltung lassen sich Präparationseigenschaften des medizinischen Instruments auf einfache Weise weiter verbessern.

Vorteilhaft ist es, wenn in einer in einer maximal angenäherten Stellung der mindestens zwei Werkzeugelemente ein Abstand der ersten Werkzeugelementfläche und der zweiten Werkzeugelementfläche voneinander konstant oder im Wesentlichen konstant ist. Beispielsweise kann so auf einfache Weise ein elektrochirurgisches Instrument realisiert werden, bei dem sich die beiden Werkzeugelementflächen zur Vermeidung eines Kurzschlusses nicht berühren dürfen.

Günstigerweise umfasst das Instrument eine Abstandshalteeinrichtung zum Vorgeben eines minimalen Abstands der mindestens zwei Werkzeugelemente voneinander in der maximal angenäherten Stellung. Mit einer solchen Abstandshalteinrichtung kann ein in Kontakt treten der Werkzeugelementflächen der Werkzeugelemente einfach und sicher verhindert werden.

Vorteilhaft ist es, wenn die Abstandshalteinrichtung mindestens ein Abstandshalteelement umfasst, welches von der ersten Werkzeugelementfläche oder von der zweiten Werkzeugelementfläche abstehend angeordnet oder ausgebildet ist. Beispielsweise können von jeder der Werkzeugelementflächen ein oder mehrere Abstandhalteelemente abstehen. Günstigerweise handelt es sich bei den Abstandshalteelementen um elektrisch nicht leitende Bauteile.

Vorzugsweise ist das mindestens eine Abstandshalteelement aus einem elektrisch isolierenden Werkstoff ausgebildet ist. Beispielsweise kann dieser Werkstoff eine Keramik sein. Elektrisch isolierend in diesem Sinne sind insbesondere Nichtleiter mit einer Leitfähigkeit im Bereich von etwa 10⁻⁸ bis über 10⁻²⁶ S/cm. Eine Keramik kann insbesondere auf einfache Weise auf einem aus einem metallischen, elektrisch leitfähigen Werkstoff ausgebildeten Werkzeugelement angebracht werden.

Vorzugsweise sind die mindestens zwei Werkzeugelemente aus einem elektrisch leitenden Werkstoff ausgebildet. Insbesondere kann es sich dabei um einen metallischen Leiter handeln. Ein solcher metallischer Leiter weist insbesondere eine elektrische Leitfähigkeit von mindestens 10⁶ S/m auf.

Die Werkzeugelemente lassen sich auf einfache Weise und mit beliebigen Formen ausbilden, wenn die mindestens zwei Werkzeugelemente in Form von Metallspritzgussteilen ausgebildet sind. Beispielsweise lassen sie sich mit einem sogenannten MIM-Verfahren herstellen. MIM steht hierbei für "Metal Injection Molding".

Gemäß einer weiteren bevorzugten Ausführungsform der Erfindung kann vorgesehen sein kann, dass die erste Werkzeugelementfläche und die zweite Werkzeugelementfläche eben ausgebildet sind. Alternativ können die erste Werkzeugelementfläche und die zweite Werkzeugelementfläche gekrümmt ausgebildet sein, insbesondere einerseits konkav und andererseits hierzu korrespondierend konvex bezogen auf die Werkzeugelementerstreckungsrichtung. So können insbesondere Werkzeugelemente ausgebildet werden, die in einer maximal angenäherten Stellung einen konstanten minimalen Abstand voneinander aufweisen, und zwar unabhängig davon, ob die Werkzeugelementflächen eben oder gekrümmt sind.

Vorteilhaft ist es, wenn das erste Werkzeugelement und das zweite Werkzeugelement jeweils einen sich in der Werkzeugelementerstreckungsrichtung erstreckenden Schlitz aufweisen, welcher die erste Werkzeugelementfläche und die zweite Werkzeugelementfläche mindestens abschnittsweise durchsetzt. Ein solcher Schlitz kann insbesondere eingesetzt werden, um ein Schneidelement einer Schneideinrichtung bei einer Bewegung von proximal kommend in distaler Richtung zu führen, um zwischen den Werkzeugelementflächen gehaltenes und miteinander durch Koagulation verbundenes Gewebe zu durchtrennen. So können beispielsweise Gefäße auf einfache Weise in einem einzigen Schritt verschweißt und durchtrennt werden.

Auf einfache Weise lassen sich die Werkzeugelemente ausbilden, wenn der Schlitz langlochartig geformt ist. Insbesondere kann der Schlitz geradlinig verlaufen, auch wenn die Werkzeugelementerstreckungsrichtung gekrümmt verläuft.

Zum Durchtrennen von Gewebe ist es insbesondere günstig, wenn das Instrument eine Schneideinrichtung umfasst mit einem Schneidelement, welches in der Werkzeugelementerstreckungsrichtung bewegbar angeordnet oder ausgebildet ist. Wie beschrieben kann so miteinander verbundenes Gewebe mit der Schneideinrichtung durchtrennt werden.

Günstig ist es, wenn das Schneidelement eine in distaler Richtung weisende Schneidkante aufweist und wenn die Schneidkante die Schlitze in den Werkzeugelementen mindestens teilweise durchgreift, wenn die Werkzeugelemente die maximal angenäherte Stellung einnehmen. Auf diese Weise kann insbesondere sichergestellt werden, dass zwischen den Werkzeugelementflächen gehaltenes Gewebe mit dem Schneidelement vollständig durchtrennt werden kann.

Um Instrumente für unterschiedlichste Einsatzzwecke ausbilden zu können, ist es vorteilhaft, wenn die Werkzeugelementerstreckungsrichtung geradlinig oder gekrümmt verläuft.

Um zu vermeiden, dass mit den Werkzeugelementen, insbesondere mit distalen Enden derselben, Gewebe und Gefäße verletzt werden können, ist es vorteilhaft, wenn distale Enden der mindestens zwei Werkzeugelemente abgerundet sind. So können sie insbesondere stumpf ausgebildet werden.

Um eine Rückhaltefunktion des Instruments noch weiter verbessern zu können, ist es günstig, wenn die Greiffläche des mindestens einen Greifelements eine strukturierte Oberfläche aufweist. Insbesondere kann die Oberfläche makroskopisch strukturiert ausgebildet sein. Hierzu kann eine Vielzahl von Vorsprüngen ausgebildet sein oder eine regelmäßige Struktur, beispielsweise in der Art eines Waffeleisens oder eines Nagelbretts.

Vorteilhaft ist es, wenn die Greiffläche des mindestens einen Greifelements mindestens teilweise, insbesondere vollständig, mit einer Beschichtung versehen ist. Die Beschichtung kann insbesondere separat ausgebildet und kraftund/oder form- und/oder stoffschlüssig mit der Greiffläche verbunden sein. Insbesondere kann die Beschichtung durch ein thermisches Beschichtungsverfahren wie beispielsweise thermisches Spritzen, chemische Gasphasenabscheidung, Flammenbeschichtung, physikalische Gasphasenabscheidung, Sputtern oder Auftragsschweißen aufgebracht sein. Die Beschichtung vorzusehen ermöglicht es beispielsweise auch mechanische Eigenschaften der Werkzeugelemente von Oberflächeneigenschaften durch eine gezielte Wahl unterschiedlicher Materialien voneinander zu trennen. Die Beschichtung optional mit strukturierter Oberfläche auszubilden ermöglicht insbesondere eine verbesserte Haftung des Gewebes.

Vorzugsweise bildet die Beschichtung die strukturierte Oberfläche. So kann insbesondere eine verbesserte Haftung des Gewebes erreicht werden. Zudem lässt sich so auch eine Strukturierung der Oberfläche einfach ausbilden.

Günstig ist es, wenn die Beschichtung in Form einer thermischen Beschichtung ausgebildet ist, insbesondere in Form einer keramischen Beschichtung. Unabhängig davon, ob die Beschichtung eine strukturierte Oberfläche aufweist oder bildet, kann so insbesondere die Gefahr einer thermischen Randschädigung der Greiffläche verringert werden und somit auch eine Schädigung umliegenden und/oder mit dem Instrument gefassten Gewebes.

Vorzugsweise ist das Instrument in Form eines elektrochirurgischen Instruments oder in Form eines Klammergeräts ausgebildet. So kann durch die Greifelemente der Einsatz des jeweiligen Instruments weiter optimiert werden. Gewebe lässt sich so einfacher zwischen den Werkzeugelementflächen platzieren und bearbeiten, beispielsweise klammern oder elektrochirurgisch durch Hochfrequenzströme verschweißen.

Insbesondere für minimalinvasive chirurgische Eingriffe ist es vorteilhaft, wenn das medizinische Instrument eine Betätigungseinrichtung umfasst, welche mit den Werkzeugelementen zusammenwirkend an einem proximalen Ende des Instruments angeordnet oder ausgebildet ist zum Bewegen derselben relativ zueinander. Beispielsweise kann die Betätigungseinrichtung über ein Kraftübertragungsglied oder über mehrere Kraftübertragungsglieder, beispielsweise Schub- und Zugglieder, die in einem Rohrschaft verschiebbar angeordnet oder ausgebildet sind, mit den Werkzeugelementen gekoppelt sein.

Die nachfolgende Beschreibung bevorzugter Ausführungsformen dient im Zusammenhang mit den Zeichnungen der näheren Erläuterung. Es zeigen:
- Figur 1:: eine perspektivische Gesamtansicht eines Ausführungsbeispiels eines medizinischen Instruments;

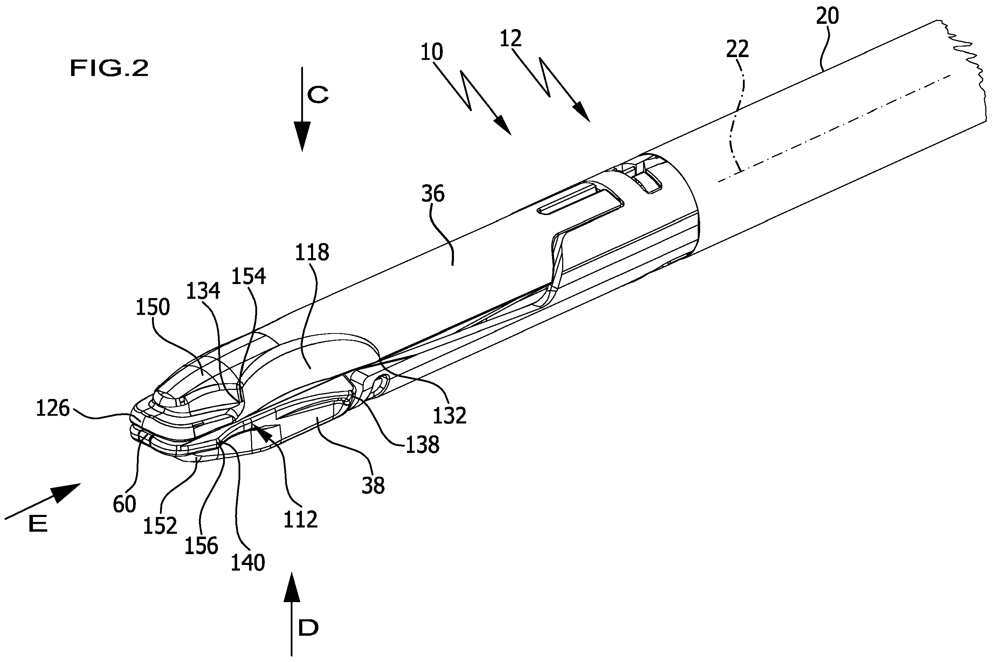

- Figur 2:: eine vergrößerte Teilansicht des Bereichs A in Figur 1;

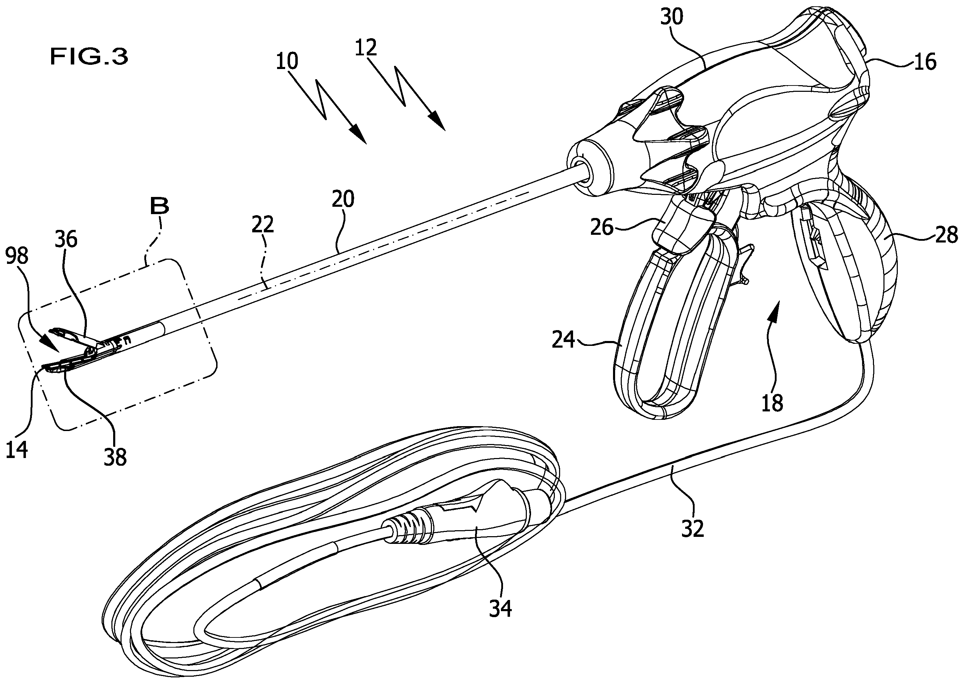

- Figur 3:: eine Ansicht ähnlich Figur 3, jedoch mit geöffnetem Instrumentenmaul;

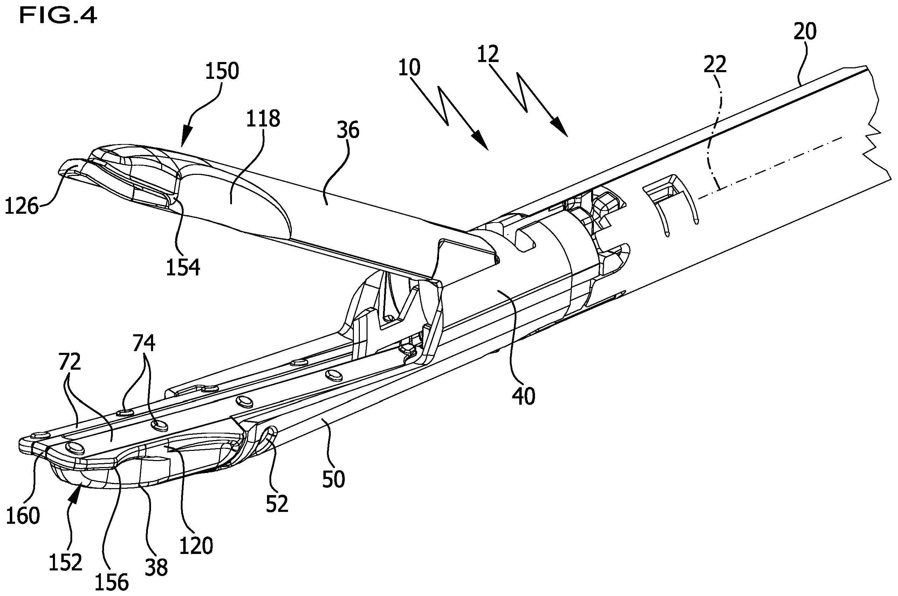

- Figur 4:: eine vergrößerte Teilansicht des Bereichs B aus Figur 3;

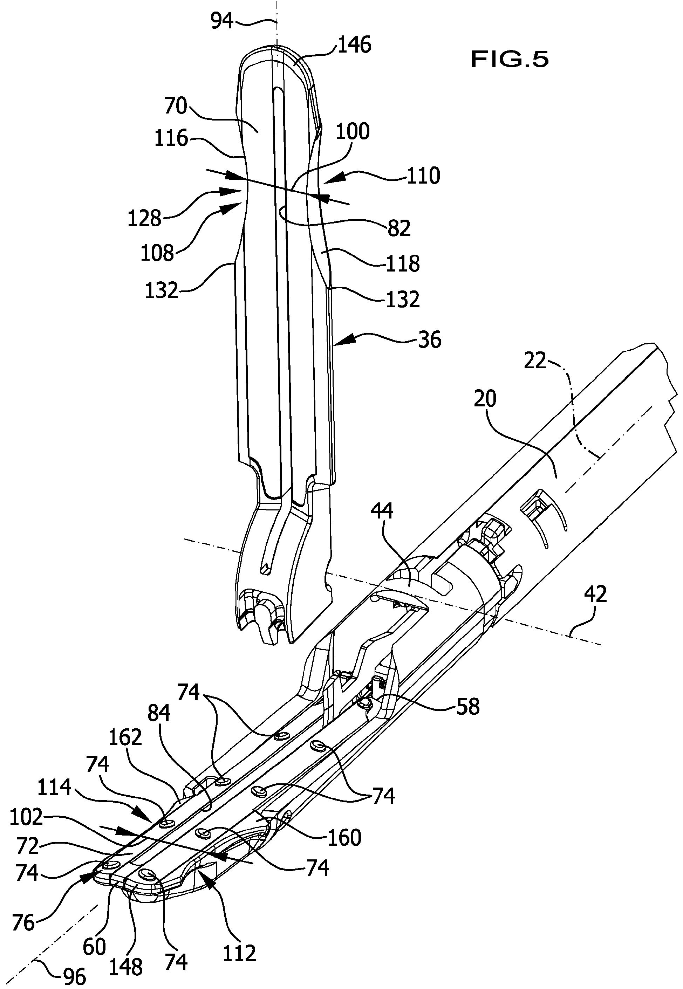

- Figur 5:: eine perspektivische, teilweise Explosionsdarstellung der Anordnung aus Figur 4;
- Figur 6:: eine Ansicht der Anordnung aus Figur 2 in Richtung des Pfeils C;
- Figur 7:: eine Ansicht der Anordnung aus Figur 2 in Richtung des Pfeils D;

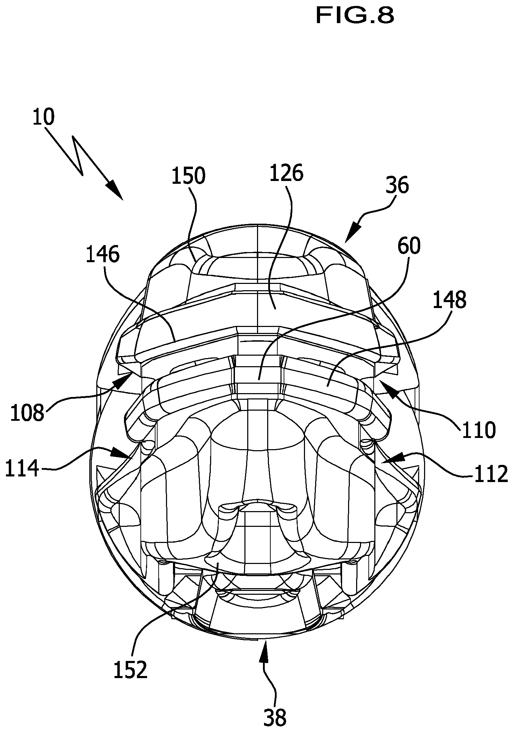

- Figur 8:: eine Ansicht der Anordnung aus Figur 2 in Richtung des Pfeils E;
- Figur 9:: eine Schnittansicht längs Linie 9-9 in Figur 6 bei geöffnetem Maul; und
- Figur 10:: eine Schnittansicht längs Linie 9-9 in Figur 6 bei geschlossenem Maul.

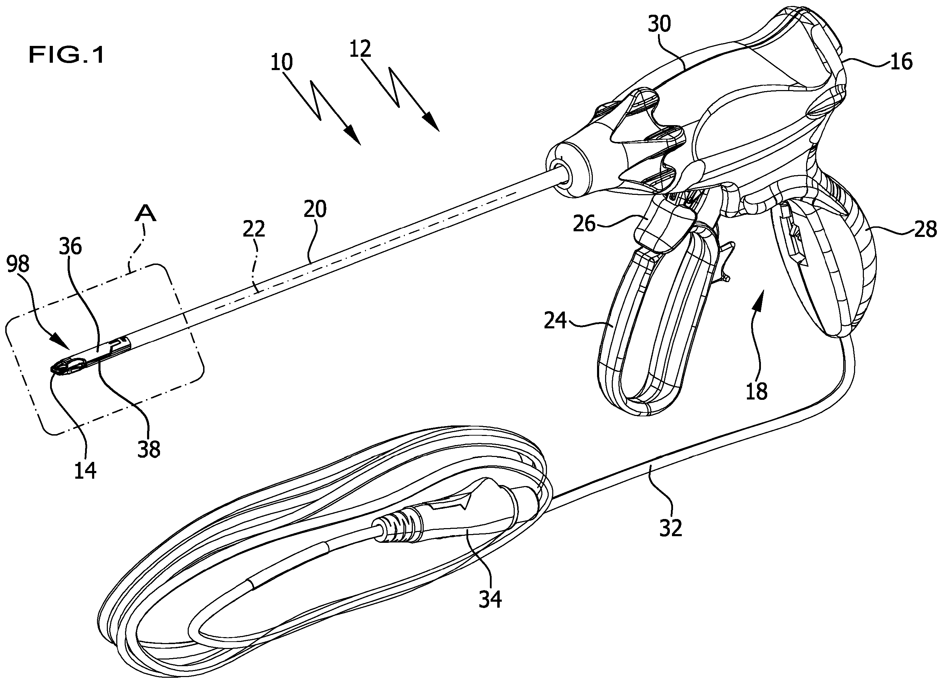

In Figur 1 ist ein Ausführungsbeispiel eines medizinischen Instruments 10 schematisch dargestellt. Es ist in Form eines elektrochirurgischen Instruments 12 ausgebildet.

Das Instrument 10 weist ein distales Ende 14 auf und definiert ein proximales Ende 16.

Am proximalen Ende 16 des Instruments 10 ist eine Betätigungseinrichtung 18 angeordnet. Diese ist relativ zu einem langgestreckten Schaft 20 um eine von diesem definierte Längsachse 22 verdrehbar angeordnet.

An der Betätigungseinrichtung 18 stehen quer zur Längsachse 22 ein erster Hebel 24 und ein zweiter Hebel 26 ab, welche in proximaler Richtung um nicht eingezeichnete, quer zur Längsachse 22 verlaufende Schwenkachsen in Richtung auf ein feststehendes Griffelement 28 verschwenkbar sind. Das Griffelement 28 steht ebenfalls quer, nahezu senkrecht, bezogen auf die Längsachse 22 von einem Grundkörper 30 der Betätigungseinrichtung 88 ab.

Aus einem freien Ende des Griffelements 28 ist ein Anschlusskabel 32 herausgeführt, an dessen freiem Ende ein Steckverbinder 34 angeordnet ist. Der Steckverbinder 34 ist ausgebildet, um mit einem korrespondierenden Steckverbinder an einer Stromversorgungseinrichtung mechanisch und elektrisch in Eingriff gebracht zu werden. So kann das Instrument 10 mit einem hochfrequenten Strom versorgt werden.

Am distalen Ende 14 des Schafts 20 sind ein erstes Werkzeugelement 36 und ein zweites Werkzeugelement 38 relativ zueinander bewegbar, nämlich verschwenkbar, angeordnet.

Das erste Werkzeugelement 36 ist an einem distalseitig aus dem Schaft 20 hervorstehenden Lagerelement 40 um eine quer zur Längsachse 22 verlaufende Schwenkachse 42 gelagert. Diese wird definiert durch einen eine Lagerwelle definierenden Querträger 44, welcher am Lagerelement 40 ausgebildet ist und mit einer rinnenförmigen Vertiefung 46 im proximalen Endbereich des ersten Werkzeugelements 36 zusammenwirkt, um dessen Verschwenkbewegung zu führen.

Das erste Werkzeugelement 36 ist über ein Kraftübertragungsglied 48 mit dem ersten Hebel 24 gekoppelt, sodass infolge einer Verschwenkbewegung des ersten Hebels 24 in Richtung auf das Griffelement 28 hin das erste Werkzeugelement 36 um die Schwenkachse 42 in Richtung auf das zweite Werkzeugelement 38 hin verschwenkt wird.

Ein unterer Teilabschnitt 50 des Lagerelements 40 erstreckt sich in distaler Richtung über den Querträger 44 hinaus und weist zwei sich quer zur Längsachse 22 erstreckende Aufnahmen 22 auf, in die quer zur Längsachse 22 vom zweiten Werkzeugelement 38 abstehende Lagerzapfen 54 eintauchen. Die Lagerzapfen 54 definieren eine weitere Schwenkachse 56, um die das zweite Werkzeugelement 38 in einem kleinen Winkelbereich verdrehbar gelagert ist.

Die Lagerzapfen 54 sind in etwa mittig zwischen einem proximalen Ende 58 und einem distalen Ende 60 des zweiten Werkzeugelements 38 angeordnet. Ein sich einerseits an einem distalen Endbereich 62 des Lagerelements 40 abstützendes Federelement 64, welches sich mit freien Enden am zweiten Werkzeugelement 38 abstützt, hält das zweite Werkzeugelement mit einer von diesem definierten Längsachse 66 bezogen auf die Längsachse 22 um einen Winkel 68 von etwa 10° in Richtung auf das erste Werkzeugelement geneigt, wenn die Werkzeugelemente 36 und 38 maximal weit voneinander weg verschwenkt sind.

Das erste Werkzeugelement 36 weist eine langgestreckt U-förmige erste Werkzeugelementfläche 70 auf, die in Richtung auf das zweite Werkzeugelement 38 hin weist. In ähnlicher Form weist das zweite Werkzeugelement 38 eine langgestreckt U-förmige zweite Werkzeugelementfläche 72 auf, die in Richtung auf das erste Werkzeugelement 36 hin weist. In einer maximal angenäherten Stellung der Werkzeugelemente 36 und 38 liegen die Werkzeugelementflächen 70 und 72 einander direkt gegenüber.

Auf der zweiten Werkzeugelementfläche 72 sind Abstandshalteelemente 74 angeordnet, die eine Abstandshalteeinrichtung 76 bilden, um einen minimalen Abstand 78 der Werkzeugelementflächen 72 und 74 in einer maximal angenäherten Stellung der Werkzeugelemente 36 und 38 vorzugeben. Bei dem in den Figuren dargestellten Ausführungsbeispiel umfasst die Abstandshalteeinrichtung 76 acht Abstandshalteelemente 74.

Die Abstandshalteelemente 74 sind aus einem elektrisch nicht leitenden Werkstoff ausgebildet. Bei dem in den Figuren dargestellten Ausführungsbeispiel sind sie aus einer Keramik ausgebildet.

Die Werkzeugelemente 36 und 38 sind aus einem elektrisch leitenden Werkstoff ausgebildet. Bei dem in den Figuren dargestellten Ausführungsbeispiel ist der elektrisch leitende Werkstoff ein metallischer Leiter.

Die Werkzeugelemente 36 und 38 sind gegeneinander elektrisch isoliert. Durch die Abstandshalteeinrichtung 76 können wie beschrieben die Werkzeugelementflächen 70 und 72 nicht miteinander in Kontakt kommen. Dies ermöglicht es, insbesondere einen Hochfrequenzstrom über die Werkzeugelemente 36 und 38 zu leiten, um beispielsweise zwischen den Werkzeugelementflächen 70 und 72 gehaltenes Gewebe oder ein Gefäß 80, wie schematisch in den Figuren dargestellt, mittels des Hochfrequenzstroms zu koagulieren und dadurch zu versiegeln.

Die Werkzeugelemente 36 und 38 sind bei dem in den Figuren dargestellten Ausführungsbeispiel jeweils mit einem Schlitz 82 beziehungsweise 84 versehen, welcher sich parallel zur Längsachse 22 erstreckt. Die Schlitze 82 und 84 durchsetzen die erste Werkzeugelementfläche 70 beziehungsweise die zweite Werkzeugelementfläche 72.

Die Schlitze 82 und 84 sind langlochartig geformt.

Das Instrument 10 umfasst ferner eine Schneideinrichtung 86 mit einem Schneidelement 88. Dieses ist parallel zur Längsachse 22 bewegbar angeordnet. Es ist über ein Kraftübertragungsglied 90, welches sich durch den Schaft 20 hindurch parallel zum ersten Kraftübertragungsglied 48 erstreckt, mit dem zweiten Hebel 26 gekoppelt.

Wird der zweite Hebel 26 in Richtung auf das Griffelement 28 hin verschwenkt, wird durch eine Bewegung des Kraftübertragungsglieds 90 in distaler Richtung das Schneidelement 88 mit seiner in distaler Richtung weisenden Schneidkante 92 bewegt.

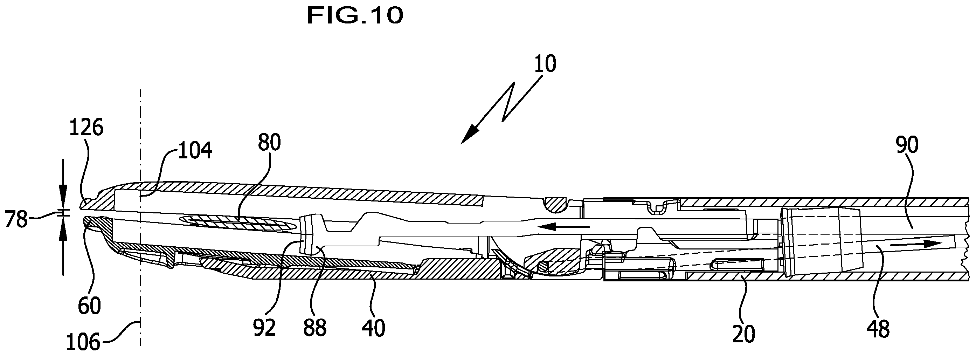

Die Schneidkante 92 greift in die beiden Schlitze 82 und 84 ein, wenn die Werkzeugelemente 36 und 38 die maximal angenäherte Stellung einnehmen. Dies ist insbesondere gut in der Schnittansicht in Figur 10 zu erkennen.

An dieser Stelle sei angemerkt, dass bei dem in den Figuren dargestellten Ausführungsbeispiel der zweite Hebel 26 nur betätigt werden kann, wenn der erste Hebel 24 in seine maximal an das Griffelement 28 angenäherte Stellung überführt ist.

Die Werkzeugelemente 36 und 38 definieren jeweils eine Werkzeugelementerstreckungsrichtung 94 beziehungsweise 96. Die Werkzeugelementerstreckungsrichtungen 94 und 96 verlaufen bei dem in den Figuren dargestellten Ausführungsbeispiel geradlinig. Sie werden definiert durch Längsachsen 97 und 66 des ersten Werkzeugelements 36 und des zweiten Werkzeugelements 38.

Ist ein durch die beiden Werkzeugelemente 36 und 38 definiertes Maul 98 geschlossen, nehmen also die beiden Werkzeugelemente 36 und 38 ihre maximal angenäherte Stellung ein, verlaufen die Werkzeugelementerstreckungsrichtungen 94 und 96 und damit die Längsachsen 97 und 66 parallel zur Längsachse 22 des Schafts 20.

Die Werkzeugelemente 36 und 38 definieren im Bereich ihrer jeweiligen Werkzeugelementflächen 70 und 72 eine Breite 100 beziehungsweise 102. Die Breite 100 erstreckt sich in einer Richtung quer, insbesondere senkrecht, zur Werkzeugelementerstreckungsrichtung 94. Die Breite 102 erstreckt sich in eine Richtung quer, insbesondere senkrecht, zur Werkzeugelementerstreckungsrichtung 96.

Die Werkzeugelementflächen 70 und 72 definieren jeweils eine Flächennormale 104 beziehungsweise 106, die senkrecht zu den Werkzeugelementflächen 70 und 72 orientiert sind.

Die Breiten 100 und 102 erstrecken sich quer, insbesondere senkrecht, zu den Flächennormalen 104 beziehungsweise 106. Die Flächennormalen 104 und 106 verlaufen parallel zueinander, wenn die Werkzeugelemente 70 und 72 ihre maximal angenäherte Stellung einnehmen.

An den Werkzeugelementen 36 und 38 sind jeweils zwei Greifelemente 108 und 110 beziehungsweise 112 und 114 angeordnet beziehungsweise ausgebildet.

Die Greifelemente 108 und 110 weisen vom ersten Werkzeugelement 36 weg und voneinander weg weisende Greifflächen 116 und 118 auf. Die Greifelemente 112 und 114 am zweiten Werkzeugelement 38 definieren voneinander weg weisende Greifflächen 120 und 122.

Die Breite 100 des ersten Werkzeugelements 36 nimmt ausgehend von dessen proximalem Ende 124 zu seinem distalen Ende 126 hin ab. Zwischen dem proximalen Ende 124 und dem distalen Ende 126 weist die Breite 100 mindestens ein lokales Minimum 128 auf. Die Breite 100 ist im lokalen Minimum minimal. Mit anderen Worten ist die Werkzeugelementfläche 70 hier am schmalsten. Die Greifflächen 116 und 118 sind im Bereich dieses lokalen Minimums der Breite 100 ausgebildet.

In ähnlicher Weise nimmt die Breite 102 des zweiten Werkzeugelements 38 ausgehend vom proximalen Ende 58 zum distalen Ende 60 hin ab. Die Breite 102 weist mindestens ein lokales Minimum 130 zwischen dem proximalen und dem distalen Ende 58, 60 auf. Die Greifflächen 120 und 122 sind im Bereich des lokalen Minimums der Breite 130 ausgebildet.

Die Greifflächen 116, 118, 120 und 122 werden in der beschriebenen Weise durch eine Art Einschnürung an den Werkzeugelementen 36 und 38 ausgebildet. So werden ihrer Art nach hakenförmige Elemente ausgebildet, und zwar beidseitig an den Werkzeugelementen 36 und 38. Mit den Greifelementen 108, 110, 112 und 114 lässt sich Gewebe oder lassen sich Gefäße zurückhalten oder zur Seite schieben, ohne dass eine große Gefahr besteht, dass das Gewebe oder die Gefäße in distaler Richtung von den Werkzeugelementen 36 und 38 abrutschen können.

Die Greifflächen 116 und 118 definieren ein proximales Ende 132 und ein distales Ende 134. Dazwischen erstreckt sich ein von den Greifflächen 116 und 118 definierter Greifbereich 136.

In ähnlicher Weise erstrecken sich die Greifflächen 120 und 122 zwischen einem proximalen Ende 138 und einem distalen Ende 140 und definieren so einen Greifbereich 142.

Das lokale Minimum 128 ist zwischen den Enden 132 und 134 angeordnet, das lokale Minimum 130 zwischen den Enden 138 und 140. Somit sind die Greifflächen 116, 118, 120 und 122 im Bereich der lokalen Minima 128 und 130 sowie voneinander weg weisend angeordnet beziehungsweise ausgebildet.

Die Greifflächen 116, 118, 120 und 122 weisen von der jeweiligen Werkzeugelementerstreckungsrichtung 94 beziehungsweise 96 weg.

Die Greifflächen 116, 118, 120 und 122 sind vom jeweiligen Werkzeugelement 36, 38 weg weisenden konkav gekrümmt. Sie weisen somit eine ähnliche Kontur auf, wie wenn die Werkzeugelemente 36 und 38 einseitig gekrümmt wären, ihre Werkzeugelementerstreckungsrichtungen 94 und 96 also nicht wie bei dem in den Figuren dargestellten Ausführungsbeispiel geradlinig verlaufen, sondern von proximal nach distal gekrümmt sind. Ein Vorteil gegenüber derart gekrümmten Maulteilen ist bei dem in den Figuren dargestellten Ausführungsbeispiel des medizinischen Instruments 10, dass so beidseitig Greifelemente 108 und 110 beziehungsweise 112 und 114 an den Werkzeugelementen 36 und 38 ausgebildet sind. Ein Operateur muss also den Schaft 20 mit dem Maul 98 nicht um 180° um die Längsachse 22 verdrehen, wenn er auf der gegenüberliegenden Seite Gewebe präparieren will. Mit dem beschriebenen Instrument 10 ist beidseitiges Präparieren und Rückhalten von Gewebe und Gefäßen möglich.

Bei dem in den Figuren dargestellten Ausführungsbeispiel des Instruments 10 bilden die Greifelemente 108, 110, 112 und 114 einen Teilbereich einer Seitenfläche der Werkzeugelemente 36 und 38.

Die Werkzeugelemente 36 und 38 weisen jeweils einen plattenförmigen Werkzeugelementbereich 146 und 148 auf, an dem jeweils die Werkzeugelementflächen 70 und 72 ausgebildet sind.

Bei dem in den Figuren dargestellten Ausführungsbeispiel definiert der plattenförmige Werkzeugelementbereich 146 beziehungsweise 148 auch die Seitenfläche des jeweiligen Werkzeugelements 36 beziehungsweise 38.

Auch die distalen Enden 126 und 60 der Werkzeugelemente 36 und 38 werden durch die plattenförmigen Werkzeugelementbereiche 146 und 148 gebildet.

Der plattenförmige Werkzeugelementbereich 146 beziehungsweise 148 trägt auf einer von der jeweiligen Werkzeugelementfläche 70 beziehungsweise 72 weg weisenden Seite eine Stützstruktur 150 beziehungsweise 152. Die Stützstrukturen 150 und 152 übergreifen insbesondere die Schlitze 82 und 84, sodass diese nur in Richtung auf das jeweils andere Werkzeugelement 36 beziehungsweise 38 hin weisend geöffnet sind, um das Schneidelement 88 teilweise aufzunehmen.

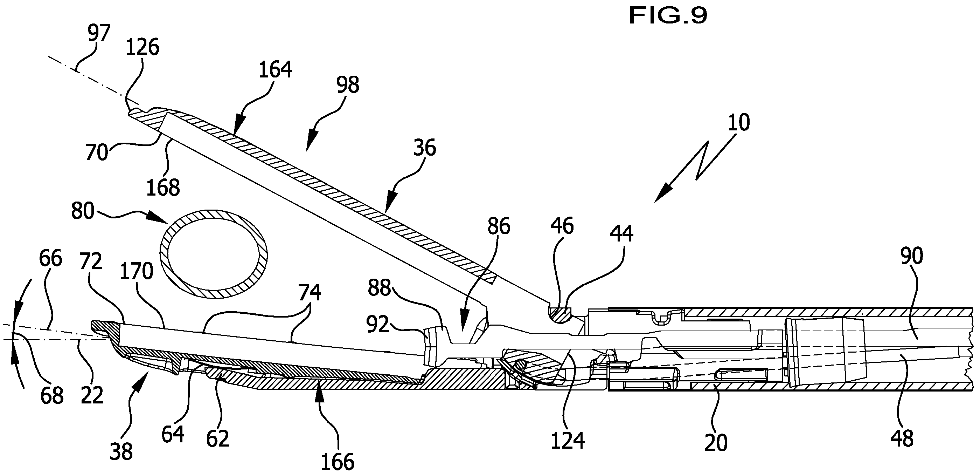

Die Stützstrukturen 150 und 152 sind bezogen auf das distale Ende 126 beziehungsweise 60 der Werkzeugelemente 36, 38 in proximaler Richtung zurückgesetzt. Damit verbleibt, wie insbesondere in Figur 9 gut zu erkennen, ein distaler Endbereich der Werkzeugelemente 36, 38, welcher ausschließlich durch einen kurzen, sich in der jeweiligen Werkzeugelementerstreckungsrichtung 94 beziehungsweise 96 erstreckenden Abschnitt gebildet wird, der ausschließlich durch ein distales Ende der plattenförmigen Werkzeugelementbereiche 146 beziehungsweise 148 gebildet wird. Die distalen Endbereiche weisen eine relativ geringe Dicke parallel zu den Flächennormalen 104 und 106 auf, sodass die distalen Enden 126 und 60 ideal zum Präparieren von Gewebe genutzt werden können. Beispielsweise lassen sie sich zwischen Gewebeschichten einschieben, um diese durch Bewegen der Werkzeugelemente 36 und 38 voneinander weg zu separieren. So kann das Gewebe durch eine Relativbewegung der Werkzeugelemente 36 und 38 auf einfache Weise gespreizt und atraumatisch präpariert werden.

Um zu verhindern, dass Gewebe oder Gefäße durch das Instrument 10 verletzt werden können, sind die distalen Enden 126 und 60 der Werkzeugelemente 36 und 38 abgerundet.

Die Greifflächen 116, 118, 120 und 122 sind tangentenstetig gekrümmt. Sie weisen also keine Ecken und Kanten auf.

Die Breite 100 beziehungsweise 102 der Werkzeugelemente 36 beziehungsweise 38 weist mindestens ein lokales Maximum 154 beziehungsweise 156 zwischen den proximalen Enden 124 beziehungsweise 58 und den distalen Enden 126 beziehungsweise 60 auf. Die Greifflächen 108, 110, 112 und 114 erstrecken sich in distaler Richtung bis zu dem lokalen Maximum 154 beziehungsweise 156. Die Maxima 154 und 156 begrenzen die Greifbereiche 136 und 142 distalseitig.

Die Breite 100 beziehungsweise 102 nimmt in distaler Richtung ausgehend vom lokalen Maximum kontinuierlich ab, bei dem in den Figuren dargestellten Ausführungsbeispiel des Instruments 10 bis zu den distalen Enden 126 und 60 der Werkzeugelemente 36 und 38.

Bei dem in den Figuren dargestellten Ausführungsbeispiel des Instruments 10 ist ein Abstand des lokalen Minimums 128 beziehungsweise 130 der Breite 100 beziehungsweise 102 vom proximalen Ende 132 beziehungsweise 138 der jeweiligen Greiffläche 116, 118, 120 und 122 größer als von einem distalen Ende 134 beziehungsweise 140 der Greiffläche 116, 118, 120 beziehungsweise 122.

Die Greifflächen 116, 118, 120 und 122 weisen bei dem in den Figuren dargestellten Ausführungsbeispiel des Instruments 10 eine strukturierte Oberfläche auf. Insbesondere ist die Oberfläche bei einem Ausführungsbeispiel makroskopisch strukturiert.

Die Greifelemente 108 und 110 beziehungsweise 112 und 114 sind am Werkzeugelement 36 beziehungsweise 38 symmetrisch angeordnet beziehungsweise ausgebildet. Die Werkzeugelemente 36 und 38 sind spiegelsymmetrisch bezogen auf eine die Längsachse 97 beziehungsweise 66 der Werkzeugelemente 36 und 38 enthaltende Spiegelebene 158 ausgebildet.

Eine Krümmung der Greifflächen 116, 118, 120 und 122 weist einen Krümmungsradius in einem Bereich von etwa 5 mm bis etwa 30 mm auf.

Die Greifflächen 116 und 118 beziehungsweise 120 und 122 sind bezogen auf die Werkzeugelementerstreckungsrichtungen 94 und 96 in distaler beziehungsweise proximaler Richtung zueinander versetzt angeordnet beziehungsweise ausgebildet. Dies äußert sich insbesondere darin, dass sich der Greifbereich 142 etwas weiter in distaler Richtung erstreckt als der Greifbereich 136.

Die Greifelemente 108, 110, 112 und 114 sind jeweils an den Werkzeugelementen 36 und 38 angeformt oder ausgebildet, und zwar einstückig im Sinne von monolithisch.

Bei einem anderen Ausführungsbeispiel eines Instruments 10 sind die Greifelemente 108, 110, 112 und 114 vom jeweiligen Werkzeugelement 36 und 38 lösbar. Eine Verbindung zwischen den Greifelementen 108, 110, 112 und 114 und den Werkzeugelementen 36 und 38 wird bei diesem Ausführungsbeispiel dadurch erreicht, dass sie in einer Verbindungsstellung kraft- und/oder formschlüssig in Eingriff stehen.

Die Greifelemente 108, 110, 112 und 114 sind näher am distalen Ende 126, 66 als am proximalen Ende 124, 64 des jeweiligen Werkzeugelements 36 beziehungsweise 38 angeordnet oder ausgebildet.

Die beschriebenen Greifflächen 116, 118, 120 und 122 der Greifelemente 108, 110, 112, und 114 verlaufen quer, insbesondere senkrecht, zu den Werkzeugelementflächen 70 beziehungsweise 72 der Werkzeugelemente 36 und 38.

Bei dem in den Figuren dargestellten Ausführungsbeispiel sind insbesondere am zweiten Werkzeugelement 38 weitere Greifflächen 160 und 162 ausgebildet, die bezogen auf die Werkzeugelementfläche 72 des Werkzeugelements 38 geneigt sind. Die Greifflächen 160 und 162 sind ebenfalls an den Greifelementen 112 und 114 ausgebildet und auch gegenüber den Greifflächen 120 und 122 geneigt.

Für eine optimale Funktionsweise des Instruments 10 ist ein Abstand der Werkzeugelementflächen 70 und 72 in einer maximal angenäherten Stellung der Werkzeugelemente 36 und 38 voneinander konstant beziehungsweise im Wesentlichen konstant.

Die Werkzeugelemente 36 und 38 bilden Klemmelemente 164 und 166. Die Werkzeugelementflächen 70 und 72 bilden Klemmflächen 168 und 170.

Die Werkzeugelemente 36 und 38 sind bei dem in den Figuren dargestellten Ausführungsbeispiel in Form von Metallspritzgussteilen ausgebildet.

Die Werkzeugelementflächen 70 und 72 sind bei dem in den Figuren dargestellten Ausführungsbeispiel gekrümmt ausgebildet, und zwar einerseits konkav und andererseits hierzu korrespondierend konvex bezogen auf die jeweilige Werkzeugelementerstreckungsrichtung 94 beziehungsweise 96.

Wie bereits beschrieben ist das Instrument 10 in Form eines elektrochirurgischen Instruments 12 ausgebildet. Alternative Ausführungsformen von Instrumenten 10 sind beispielsweise in Form von Klammergeräten ausgebildet. Bei diesen müssen die Werkzeugelemente nicht relativ zueinander elektrisch isoliert sein. Derartige Klammergeräte sind insbesondere ausgebildet, um chirurgische Clips zum Verbinden von Gewebeteilen zu applizieren.

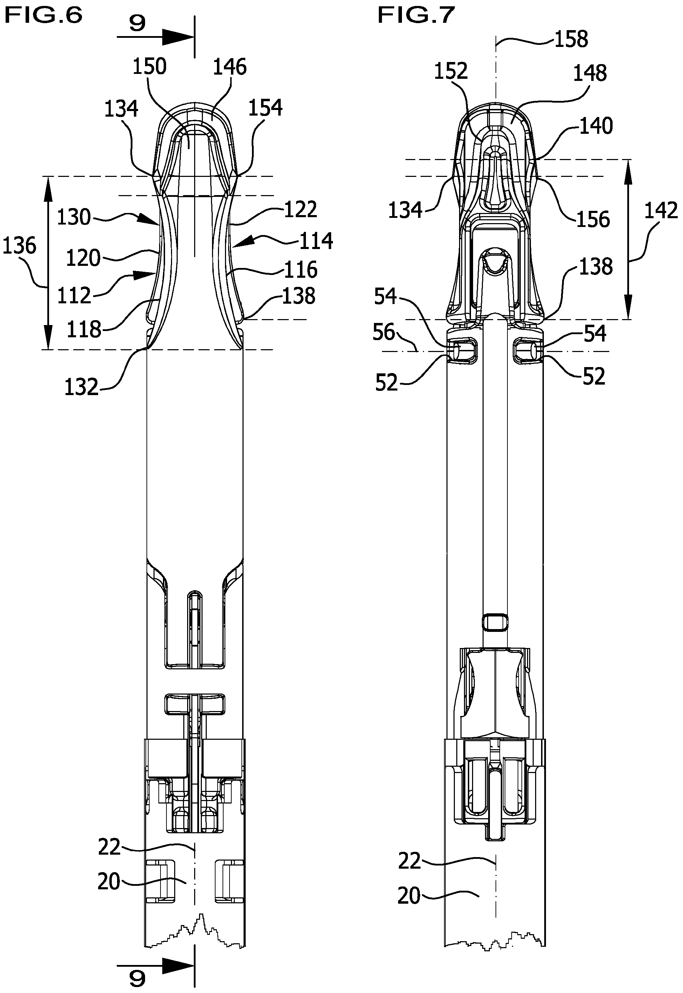

Überdies ist bei weiteren, in den Figuren nicht dargestellten Ausführungsbeispielen von Instrumenten die besondere Ausgestaltung der Werkzeugelemente mit Greifelementen grundsätzlich auch bei anderen Instrumententypen denkbar. Insbesondere kommen hierfür alle Arten von Instrumenten infrage, die an ihrem distalen Ende mindestens ein Werkzeugelement aufweisen. Die Handhabung und Präparierung von Gewebe mit dem Maul 98 ist so auf einfache Weise möglich. Dies ergibt sich sofort und unmittelbar aus den Figuren 6 und 7, in welchen die Greifbereiche 136 und 142 gut zu erkennen sind, die ein beidseitiges Präparieren von Gewebe im Bereich eines Operationssitus ermöglichen, ohne dass das Maul 98 insgesamt gekrümmt ist.

Insgesamt lässt sich mit dem beschriebenen Instrument 10 Gewebe im Maul 98 einfach platzieren. Zudem ist das distale Ende 14 des Instruments deutlich besser sichtbar als dies bei herkömmlichen Instrumenten der Fall, insbesondere aufgrund der sich ergebenden markanten Form des Mauls 98 aufgrund der beschriebenen Greifelemente 108, 110, 112 und 114.

### Bezugszeichenliste

- 10: Instrument
- 12: Instrument
- 14: Ende
- 16: Ende
- 18: Betätigungseinrichtung
- 20: Schaft
- 22: Längsachse
- 24: erster Hebel
- 26: zweiter Hebel
- 28: Griffelement
- 30: Grundkörper
- 32: Anschlusskabel
- 34: Steckverbinder
- 36: erstes Werkzeugelement
- 38: zweites Werkzeugelement
- 40: Lagerelement
- 42: Schwenkachse
- 44: Querträger
- 46: Vertiefung
- 48: Kraftübertragungsglied
- 50: Teilabschnitt
- 52: Aufnahme
- 54: Lagerzapfen
- 56: Schwenkachse
- 58: Ende
- 60: Ende
- 62: Endbereich
- 64: Federelement
- 66: Längsachse
- 68: Winkel
- 70: erste Werkzeugelementfläche
- 72: zweite Werkzeugelementfläche
- 74: Abstandshalteelement
- 76: Abstandshalteeinrichtung
- 78: Abstand
- 80: Gefäß
- 82: Schlitz
- 84: Schlitz
- 86: Schneideinrichtung
- 88: Schneidelement
- 90: Kraftübertragungsglied
- 92: Schneidkante
- 94: Werkzeugelementerstreckungsrichtung
- 96: Werkzeugelementerstreckungsrichtung
- 97: Längsachse
- 98: Maul
- 100: Breite
- 102: Breite
- 104: Flächennormale
- 106: Flächennormale
- 108: Greifelement
- 110: Greifelement
- 112: Greifelement
- 114: Greifelement
- 116: Greiffläche
- 118: Greiffläche
- 120: Greiffläche
- 122: Greiffläche
- 124: Ende
- 126: Ende
- 128: Minimum
- 130: Minimum
- 132: Ende
- 134: Ende
- 136: Greifbereich
- 138: Ende
- 140: Ende
- 142: Greifbereich
- 146: Werkzeugelementbereich
- 148: Werkzeugelementbereich
- 150: Stützstruktur
- 152: Stützstruktur
- 154: Maximum
- 156: Maximum
- 158: Spiegelebene
- 160: Greiffläche
- 162: Greiffläche
- 164: Klemmelement
- 166: Klemmelement
- 168: Klemmfläche
- 170: Klemmfläche

## Patentansprüche

1. Medizinisches Instrument (10) mit einem distalen und einem proximalen Ende (14, 16), wobei am distalen Ende (14) ein erstes Werkzeugelement (36) und ein zweites Werkzeugelement (38) relativ zueinander bewegbar angeordnet oder ausgebildet sind, wobei die Werkzeugelemente (36, 38) eine Werkzeugelementerstreckungsrichtung (94, 96) von proximal nach distal definieren, wobei das erste Werkzeugelement (36) eine erste Werkzeugelementfläche (70) und das zweite Werkzeugelement (38) eine zweite Werkzeugelementfläche (72) definieren, wobei die erste Werkzeugelementfläche (70) und die zweite Werkzeugelementfläche (72) aufeinander zu weisen, wobei die Werkzeugelemente (36, 38) im Bereich ihrer Werkzeugelementflächen (70, 72) eine Breite (100, 102) definieren, welche sich in einer Richtung quer, insbesondere senkrecht, zur Werkzeugelementerstreckungsrichtung (94, 96) und quer, insbesondere senkrecht, zu einer Flächennormalen (104, 106) der ersten Werkzeugelementfläche (70) und/oder der zweiten Werkzeugelementfläche (72) erstreckt, wobei das erste Werkzeugelement (36) und/oder das zweite Werkzeugelement (38) mindestens ein Greifelement (108, 110, 112, 114) mit einer vom jeweiligen Werkzeugelement (36, 38) weg weisenden Greiffläche (116, 118) aufweist, wobei die Breite (100, 102) des ersten und/oder zweiten Werkzeugelements (36, 38) ausgehend vom proximalen Ende (124, 58) desselben zum distalen Ende (126, 60) desselben hin abnimmt, wobei die Breite (100, 102) mindestens ein lokales Minimum (128, 130) zwischen dem proximalen und dem distalen Ende (124, 126, 58, 60) aufweist und wobei die Greiffläche (116, 118, 120, 122) im Bereich des lokalen Minimums (128, 130) der Breite (100, 102) ausgebildet ist, wobei das erste Werkzeugelement (36) und/oder das zweite Werkzeugelement (38) einen plattenförmigen Werkzeugelementbereich (146, 148) umfassen und wobei die Werkzeugelementfläche (70, 72) am plattenförmigen Werkzeugelementbereich (146, 148) des jeweiligen Werkzeugelements (36, 38) ausgebildet ist, **dadurch gekennzeichnet, dass** das distale Ende (126, 60) des jeweiligen Werkzeugelements (36, 38) durch den plattenförmigen Werkzeugelementbereich (146, 148) gebildet ist.

2. Medizinisches Instrument nach Anspruch 1, **dadurch gekennzeichnet, dass** das erste Werkzeugelement (36) und/oder das zweite Werkzeugelement (38) zwei Greifelemente (108, 110, 112, 114) umfassen, wobei insbesondere die zwei Greifelemente (108, 110, 112, 114)
a) symmetrisch am jeweiligen Werkzeugelement (36, 38) angeordnet oder ausgebildet sind
und/oder
b) jeweils eine Greiffläche (116, 118, 120, 122) definieren, die im Bereich des lokalen Minimums (128, 130) der Breite (100, 102) und voneinander weg weisend angeordnet oder ausgebildet sind.

3. Medizinisches Instrument nach einem der voranstehenden Ansprüche, **dadurch gekennzeichnet, dass**
a) die Greiffläche (116, 118, 120, 122) von der
Werkzeugelementerstreckungsrichtung (94, 96) weg weist und/oder
b) das erste Werkzeugelement (36) und das zweite Werkzeugelement (38) in Form von Klemmelementen (164, 166) ausgebildet sind und dass die erste Werkzeugelementfläche (70) und die zweite Werkzeugelementfläche (72) in Form von Klemmflächen (168, 170) ausgebildet sind
und/oder
c) die Greiffläche (116, 118, 120, 122) des mindestens einen Werkzeugelements (36, 38) vom jeweiligen Werkzeugelement (36, 38) weg weisend konkav gekrümmt ist
und/oder
d) das mindestens eine Greifelement (108, 110, 112, 114) einen Teilbereich einer Seitenfläche des jeweiligen Werkzeugelements (36, 38) umfasst.

4. Medizinisches Instrument nach einem der voranstehenden Ansprüche, **dadurch gekennzeichnet, dass**
a) der plattenförmige Werkzeugelementbereich (146, 148) die Seitenfläche des jeweiligen Werkzeugelements (36, 38) definiert
und/oder
b) der plattenförmige Werkzeugelementbereich (146, 148) auf einer von der jeweiligen Werkzeugelementfläche (70, 72) weg weisenden Seite eine Stützstruktur (150, 152) trägt,
wobei insbesondere die Stützstruktur (150, 152) bezogen auf das distale Ende (126, 60) des Werkzeugelements (36, 38) in proximaler Richtung zurückgesetzt ist.

5. Medizinisches Instrument nach einem der voranstehenden Ansprüche, **dadurch gekennzeichnet, dass**
a) die Greiffläche (116, 118, 120, 122) des mindestens einen Greifelements tangentenstetig gekrümmt ist
und/oder
b) die Breite (100, 102) mindestens ein lokales Maximum (154, 156) zwischen dem proximalen und dem distalen Ende (124, 126, 58, 60) aufweist und dass sich die Greiffläche (108, 110, 112, 114) in distaler Richtung bis zu dem lokalen Maximum erstreckt,
wobei insbesondere die Breite (100, 102) in distaler Richtung ausgehend vom lokalen Maximum (154, 156) kontinuierlich abnimmt.

6. Medizinisches Instrument nach einem der voranstehenden Ansprüche, **dadurch gekennzeichnet, dass** ein Abstand des lokalen Minimums (128, 130) der Breite (100, 102) von einem proximalen Ende (132, 138) der Greiffläche (116, 118, 120, 122) größer ist als von einem distalen Ende (134, 140) der Greiffläche (116, 118, 120, 122).

7. Medizinisches Instrument nach einem der voranstehenden Ansprüche, **dadurch gekennzeichnet, dass**
a) das erste Werkzeugelement (36) und/oder das zweite Werkzeugelement (38) spiegelsymmetrisch bezogen auf eine die Werkzeugelementerstreckungsrichtung (94, 96) definierende Längsachse (97, 66) des jeweiligen Werkzeugelements (36, 38) enthaltende Spiegelebene (158) ausgebildet sind
und/oder
b) eine Krümmung der Greiffläche (116, 118, 120, 122) des mindestens einen Greifelements (108, 110, 112, 114) einen Krümmungsradius in einem Bereich von etwa 5 mm bis etwa 30 mm definiert, insbesondere etwa 15 mm,
und/oder
c) die Greiffläche (116, 118) des mindestens einen Greifelements (108, 110) am ersten Werkzeugelement (36) und die Greiffläche (120, 122) des mindestens einen Greifelements (112, 114) am zweiten Werkzeugelement (38) in der Werkzeugelementerstreckungsrichtung (94, 96) überlappend oder in distaler oder proximaler Richtung zueinander versetzt angeordnet oder ausgebildet sind.

8. Medizinisches Instrument nach einem der voranstehenden Ansprüche, **dadurch gekennzeichnet, dass** das mindestens eine Greifelement (108, 110, 112, 114)
a) einstückig am jeweiligen Werkzeugelement (36, 38) angeformt oder ausgebildet ist
oder
b) mit dem jeweiligen Werkzeugelement (36, 38) lösbar verbindbar ausgebildet ist,
wobei insbesondere das mindestens eine Greifelement (108, 110, 112, 114) und das jeweilige Werkzeugelement (36, 38) in einer Verbindungsstellung kraft- und/oder formschlüssig in Eingriff stehen.

9. Medizinisches Instrument nach einem der voranstehenden Ansprüche, **dadurch gekennzeichnet, dass**
a) das mindestens eine Greifelement (108, 110, 112, 114) näher am distalen Ende (126, 66) als am proximalen Ende (124, 64) des jeweiligen Werkzeugelements (36, 38) angeordnet oder ausgebildet ist
und/oder
b) die Greiffläche (116, 118, 120, 122) des mindestens einen Greifelements (108, 110, 112, 114) quer, insbesondere senkrecht, zur Werkzeugelementfläche (70, 72) des jeweiligen Werkzeugelements (36, 38) verläuft
und/oder
c) das mindestens eine Greifelement (112, 114) eine weitere Greiffläche (160, 162) aufweist und dass die weitere Greiffläche bezogen auf die Werkzeugelementfläche (72) des jeweiligen Werkzeugelements (38) geneigt ist
und/oder
d) in einer maximal angenäherten Stellung der mindestens zwei Werkzeugelemente (36, 38) ein Abstand der ersten Werkzeugelementfläche (70) und der zweiten Werkzeugelementfläche (72) voneinander konstant oder im Wesentlichen konstant ist,
wobei insbesondere das Instrument (10) eine Abstandshalteeinrichtung (76) umfasst zum Vorgeben eines minimalen Abstands (78) der mindestens zwei Werkzeugelemente (36, 38) voneinander in der maximal angenäherten Stellung,
wobei weiter insbesondere die Abstandshalteeinrichtung (76) mindestens ein Abstandhalteelement (74) umfasst, welches von der ersten Werkzeugelementfläche (70) oder von der zweiten Werkzeugelementfläche (72) abstehend angeordnet oder ausgebildet ist,
wobei weiter insbesondere das mindestens eine Abstandshalteelement (74) aus einem elektrisch isolierenden Werkstoff ausgebildet ist, insbesondere aus einer Keramik.

10. Medizinisches Instrument nach einem der voranstehenden Ansprüche, **dadurch gekennzeichnet, dass** die mindestens zwei Werkzeugelemente (36, 38)
a) aus einem elektrisch leitenden Werkstoff ausgebildet sind, insbesondere aus einem metallischen Leiter,
und/oder
b) in Form von Metallspritzgussteilen ausgebildet sind.

11. Medizinisches Instrument nach einem der voranstehenden Ansprüche, **dadurch gekennzeichnet, dass** die erste Werkzeugelementfläche (70) und die zweite Werkzeugelementfläche (72) eben oder dass die erste Werkzeugelementfläche (70) und die zweite Werkzeugelementfläche (72) gekrümmt ausgebildet sind, insbesondere einerseits konkav und andererseits hierzu korrespondierend konvex bezogen auf die Werkzeugelementerstreckungsrichtung (94, 96).

12. Medizinisches Instrument nach einem der voranstehenden Ansprüche, **dadurch gekennzeichnet, dass** das erste Werkzeugelement (36) und das zweite Werkzeugelement (38) jeweils einen sich in der Werkzeugelementerstreckungsrichtung (94, 96) erstreckenden Schlitz (82, 84) aufweisen, welcher die erste Werkzeugelementfläche (70) und die zweite Werkzeugelementfläche (72) mindestens abschnittsweise durchsetzt, wobei insbesondere der Schlitz (82, 84) langlochartig geformt ist.

13. Medizinisches Instrument nach einem der voranstehenden Ansprüche, **dadurch gekennzeichnet, dass** das Instrument
a) eine Schneideinrichtung (86) umfasst mit einem Schneidelement (88), welches in der Werkzeugelementerstreckungsrichtung (94, 96) bewegbar angeordnet oder ausgebildet ist,
wobei insbesondere das erste Werkzeugelement (36) und das zweite Werkzeugelement (38) jeweils einen sich in der Werkzeugelementerstreckungsrichtung (94, 96) erstreckenden Schlitz (82, 84) aufweisen, welcher die erste Werkzeugelementfläche (70) und die zweite Werkzeugelementfläche (72) mindestens abschnittsweise durchsetzt, wobei das Schneidelement (88) eine in distaler Richtung weisende Schneidkante (92) aufweist und wobei die Schneidkante die Schlitze (82, 84) in den Werkzeugelementen (36, 38) mindestens teilweise durchgreift, wenn die Werkzeugelemente (36, 38) die maximal angenäherte Stellung einnehmen,
und/oder
b) das Instrument (10) in Form eines elektrochirurgischen Instruments (12) oder in Form eines Klammergeräts ausgebildet ist und/oder
c) das Instrument eine Betätigungseinrichtung (18) umfasst, welche mit den Werkzeugelementen (36, 38) zusammenwirkend an einem proximalen Ende (16) des Instruments (10) angeordnet oder ausgebildet ist zum Bewegen derselben relativ zueinander.

14. Medizinisches Instrument nach einem der voranstehenden Ansprüche, **dadurch gekennzeichnet, dass**
a) die Werkzeugelementerstreckungsrichtung (94, 96) geradlinig oder gekrümmt verläuft
und/oder
b) distale Enden (126, 60) der mindestens zwei Werkzeugelemente (36, 38) abgerundet sind.

15. Medizinisches Instrument nach einem der voranstehenden Ansprüche, **dadurch gekennzeichnet, dass** die Greiffläche (116, 118, 120, 122) des mindestens einen Greifelements (108, 110, 112, 114)
a) eine strukturierte Oberfläche aufweist, insbesondere eine makroskopisch strukturierte Oberfläche
und/oder
b) mindestens teilweise, insbesondere vollständig, mit einer Beschichtung versehen ist,
wobei insbesondere die Beschichtung
- die strukturierte Oberfläche bildet
und/oder
- in Form einer thermischen Beschichtung ausgebildet ist, insbesondere in Form einer keramischen Beschichtung.

## Claims

1. Medical instrument (10) with a distal and a proximal end (14, 16), wherein a first tool element (36) and a second tool element (38) are arranged or formed on the distal end (14) so as to be movable relative to one another, wherein the tool elements (36, 38) define a tool element direction of extent (94, 96) from proximal to distal, wherein the first tool element (36) defines a first tool element face (70) and the second tool element (38) defines a second tool element face (72), wherein the first tool element face (70) and the second tool element face (72) face toward one another, wherein the tool elements (36, 38) define in the region of their tool element faces (70, 72) a width (100, 102), which extends in a direction transverse, in particular perpendicular, to the tool element direction of extent (94, 96) and transverse, in particular perpendicular, to a surface normal (104, 106) of the first tool element face (70) and/or the second tool element face (72), wherein the first tool element (36) and/or the second tool element (38) has at least one gripping element (108, 110, 112, 114) with a gripping face (116, 118) facing away from the respective tool element (36, 38), wherein the width (100, 102) of the first and/or second tool element (36, 38) decreases commencing from the proximal end (124, 58) thereof toward the distal end (126, 60) thereof, wherein the width (100, 102) has at least one local minimum (128, 130) between the proximal and the distal end (124, 126, 58, 60), and wherein the gripping face (116, 118, 120, 122) is formed in the region of the local minimum (128, 130) of the width (100, 102), wherein the first tool element (36) and/or the second tool element (38) comprise(s) a plate-shaped tool element region (146, 148) and wherein the tool element face (70, 72) is formed on the plate-shaped tool element region (146, 148) of the respective tool element (36, 38), **characterized in that** the distal end (126, 60) of the respective tool element (36, 38) is formed by the plate-shaped tool element region (146, 148).

2. Medical instrument in accordance with Claim 1, **characterized in that** the first tool element (36) and/or the second tool element (38) comprise(s) two gripping elements (108, 110, 112, 114),
wherein, in particular, the two gripping elements (108, 110, 112, 114)
a) are arranged or formed symmetrically on the respective tool element (36, 38)
and/or
b) each define a gripping face (116, 118, 120, 122), which are arranged or formed in the region of the local minimum (128, 130) of the width (100, 102) and facing away from one another.

3. Medical instrument in accordance with any one of the preceding Claims, **characterized in that**
a) the gripping face (116, 118, 120, 122) faces away from the tool element direction of extent (94, 96)
and/or
b) the first tool element (36) and the second tool element (38) are configured in the form of clamping elements (164, 166) and **in that** the first tool element face (70) and the second tool element face (72) are configured in the form of clamping faces (168, 170)
and/or
c) the gripping face (116, 118, 120, 122) of the at least one tool element (36, 38) is concavely curved facing away from the respective tool element (36, 38)
and/or
d) the at least one gripping element (108, 110, 112, 114) comprises a partial region of a side face of the respective tool element (36, 38).

4. Medical instrument in accordance with any one of the preceding Claims, **characterized in that**
a) the plate-shaped tool element region (146, 148) defines the side face of the respective tool element (36, 38)
and/or
b) the plate-shaped tool element region (146, 148) bears a support structure (150, 152) on a side facing away from the respective tool element face (70, 72),
wherein, in particular, the support structure (150, 152) is recessed in the proximal direction in relation to the distal end (126, 60) of the tool element (36, 38).

5. Medical instrument in accordance with any one of the preceding Claims, **characterized in that**
a) the gripping face (116, 118, 120, 122) of the at least one gripping element is curved in a tangentially continuous manner
and/or
b) the width (100, 102) has at least one local maximum (154, 156) between the proximal and the distal end (124, 126, 58, 60) and **in that** the gripping face (108, 110, 112, 114) extends in the distal direction up to the local maximum,
wherein, in particular, the width (100, 102) continuously decreases in the distal direction commencing from the local maximum (154, 156).

6. Medical instrument in accordance with any one of the preceding Claims, **characterized in that** a distance of the local minimum (128, 130) of the width (100, 102) from a proximal end (132, 138) of the gripping face (116, 118, 120, 122) is greater than from a distal end (134, 140) of the gripping face (116, 118, 120, 122).

7. Medical instrument in accordance with any one of the preceding Claims, **characterized in that**
a) the first tool element (36) and/or the second tool element (38) are of mirror-symmetrical configuration with respect to a mirror plane (158) containing the longitudinal axis (97, 66) of the respective tool element (36, 38) defining the tool element direction of extent (94, 96)
and/or
b) a curvature of the gripping face (116, 118, 120, 122) of the at least one gripping element (108, 110, 112, 114) defines a radius of curvature in a range of about 5 mm to about 30 mm, in particular about 15 mm,
and/or
c) the gripping face (116, 118) of the at least one gripping element (108, 110) on the first tool element (36) and the gripping face (120, 122) of the at least one gripping element (112, 114) on the second tool element (38) are arranged or formed overlapping in the tool element direction of extent (94, 96) or offset from one another in the distal or proximal direction.

8. Medical instrument in accordance with any one of the preceding Claims, **characterized in that** the at least one gripping element (108, 110, 112, 114)
a) is molded or formed in one piece on the respective tool element (36, 38)
or
b) is configured to be releasably connectable to the respective tool element (36, 38),
wherein, in particular, the at least one gripping element (108, 110, 112, 114) and the respective tool element (36, 38) are in forcelocking and/or positive-locking engagement in a connecting position.

9. Medical instrument in accordance with any one of the preceding Claims, **characterized in that**
a) the at least one gripping element (108, 110, 112, 114) is arranged or formed closer to the distal end (126, 66) than to the proximal end (124, 64) of the respective tool element (36, 38)
and/or
b) the gripping face (116, 118, 120, 122) of the at least one gripping element (108, 110, 112, 114) extends transversely, in particular perpendicularly, to the tool element face (70, 72) of the respective tool element (36, 38)
and/or
c) the at least one gripping element (112, 114) has a further gripping face (160, 162) and **in that** the further gripping face is inclined in relation to the tool element face (72) of the respective tool element (38)
and/or
d) in a maximally proximate position of the at least two tool elements (36, 38), a distance of the first tool element face (70) and the second tool element face (72) from one another is constant or substantially constant,
wherein, in particular, the instrument (10) comprises a spacer device (76) for specifying a minimum distance (78) of the at least two tool elements (36, 38) from one another in the maximally proximate position,
wherein, further in particular, the spacer device (76) comprises at least one spacer element (74), which is arranged or formed projecting from the first tool element face (70) or from the second tool element face (72),
wherein, further in particular, the at least one spacer element (74) is made of an electrically insulating material, in particular of a ceramic.

10. Medical instrument in accordance with any one of the preceding Claims, **characterized in that** the at least two tool elements (36, 38)
a) are made of an electrically conductive material, in particular of a metallic conductor,
and/or
b) are configured in the form of metal injection molded parts.

11. Medical instrument in accordance with any one of the preceding Claims, **characterized in that** the first tool element face (70) and the second tool element face (72) is planar or **in that** the first tool element face (70) and the second tool element face (72) are of curved configuration, in particular concavely on the one hand and convexly corresponding thereto on the other hand in relation to the tool element direction of extent (94, 96).

12. Medical instrument in accordance with any one of the preceding Claims, **characterized in that** the first tool element (36) and the second tool element (38) each have a slit (82, 84) extending in the tool element direction of extent (94, 96), which slit passes through the first tool element face (70) and the second tool element face (72) at least in sections,
wherein, in particular, the slit (82, 84) is shaped like an oblong hole.

13. Medical instrument in accordance with any one of the preceding Claims, **characterized in that** the instrument
a) comprises a cutting device (86) with a cutting element (88), which is arranged or formed so as to be movable in the tool element direction of extent (94, 96),
wherein, in particular, the first tool element (36) and the second tool element (38) each have a slit (82, 84) extending in the tool element direction of extent (94, 96), which slit passes through the first tool element face (70) and the second tool element face (72) at least in sections, wherein the cutting element (88) has a cutting edge (92) pointing in the distal direction, and wherein the cutting edge at least partially passes through the slits (82, 84) in the tool elements (36, 38) when the tool elements (36, 38) adopt the maximally proximate position,
and/or
b) the instrument (10) is configured in the form of an electrosurgical instrument (12) or in the form of a stapler
and/or
c) the instrument comprises an actuating device (18), which is arranged or formed cooperating with the tool elements (36, 38) on a proximal end (16) of the instrument (10) for moving same relative to one another.

14. Medical instrument in accordance with any one of the preceding Claims, **characterized in that**
a) the tool element direction of extent (94, 96) extends in a rectilinear or curved manner
and/or
b) distal ends (126, 60) of the at least two tool elements (36, 38) are rounded.

15. Medical instrument in accordance with any one of the preceding Claims, **characterized in that** the gripping face (116, 118, 120, 122) of the at least one gripping element (108, 110, 112, 114)
a) has a structured surface, in particular a macroscopically structured surface,
and/or
b) is at least partially, in particular completely, provided with a coating,
wherein, in particular, the coating
- forms the structured surface
and/or
- is configured in the form of a thermal coating, in particular in the form of a ceramic coating.

## Revendications

1. Instrument médical (10) comportant une extrémité distale et une extrémité proximale (14, 16), un premier élément d'outil (36) et un deuxième élément d'outil (38) étant agencés ou réalisés de manière mobile l'un par rapport à l'autre à l'extrémité distale (14), les éléments d'outil (36, 38) définissant une direction d'extension des éléments d'outil (94, 96) de proximal vers distal, le premier élément d'outil (36) définissant une première surface d'élément d'outil (70) et le deuxième élément d'outil (38) définissant une deuxième surface d'élément d'outil (72), la première surface d'élément d'outil (70) et la deuxième surface d'élément d'outil (72) étant tournées l'une vers l'autre, les éléments d'outil (36, 38) définissant, dans la zone de leurs surfaces d'élément d'outil (70, 72), une largeur (100, 102) qui s'étend dans une direction transversale, en particulier perpendiculaire, à la direction d'extension des éléments d'outil (94, 96) et transversalement, en particulier perpendiculairement, à une normale à la surface (104, 106) de la première surface d'élément d'outil (70) et/ou de la deuxième surface d'élément d'outil (72), le premier élément d'outil (36) et/ou le deuxième élément d'outil (38) présentant au moins un élément de préhension (108, 110, 112, 114) avec une surface de préhension (116, 118) orientée à l'opposé de l'élément d'outil (36, 38) correspondant, la largeur (100, 102) du premier et/ou du deuxième élément d'outil (36, 38) diminuant à partir de l'extrémité proximale (124, 58) de celui-ci vers l'extrémité distale (126, 60) de celui-ci, la largeur (100, 102) présentant au moins un minimum local (128, 130) entre les extrémités proximale et distale (124, 126, 58, 60) et la surface de préhension (116, 118, 120, 122) étant formée dans la zone du minimum local (128, 130) de la largeur (100, 102), le premier élément d'outil (36) et/ou le deuxième élément d'outil (38) comprenant une zone d'élément d'outil en forme de plaque (146, 148) et la surface d'élément d'outil (70, 72) étant formée sur la zone d'élément d'outil en forme de plaque (146, 148) de l'élément d'outil respectif (36, 38), **caractérisé en ce que** l'extrémité distale (126, 60) de l'élément d'outil respectif (36, 38) est formée par la zone d'élément d'outil en forme de plaque (146, 148).

2. Instrument médical selon la revendication 1, **caractérisé en ce que** le premier élément d'outil (36) et/ou le deuxième élément d'outil (38) comprennent deux éléments de préhension (108, 110, 112, 114), où, en particulier, les deux éléments de préhension (108, 110, 112, 114)
a) étant agencés ou formés symétriquement sur l'élément d'outil correspondant (36, 38)
et/ou
b) définissant chacun une surface de préhension (116, 118, 120, 122), les surfaces de préhension étant agencées dans la zone du minimum local (128, 130) de la largeur (100, 102) et à distance l'une de l'autre ou formées de manière à être agencées ainsi.

3. Instrument médical selon l'une des revendications précédentes, **caractérisé en ce que**
a) la surface de préhension (116, 118, 120, 122) est orientée à l'opposé de la direction d'extension des éléments d'outil (94, 96) et/ou
b) le premier élément d'outil (36) et le deuxième élément d'outil (38) sont réalisés sous forme d'éléments de serrage (164, 166) et que la première surface d'élément d'outil (70) et la deuxième surface d'élément d'outil (72) sont réalisées sous forme de surfaces de serrage (168, 170)
et/ou
c) la surface de préhension (116, 118, 120, 122) du ou des éléments d'outil (36, 38) est incurvée de manière concave en s'éloignant de l'élément d'outil correspondant (36, 38)
et/ou
d) le ou les éléments de préhension (108, 110, 112, 114) comprennent une partie d'une surface latérale de l'élément d'outil respectif (36, 38).

4. Instrument médical selon l'une des revendications précédentes,
**caractérisé en ce que**
a) la zone d'élément d'outil en forme de plaque (146, 148) définit la surface latérale de l'élément d'outil correspondant (36, 38)
et/ou
b) la zone d'élément d'outil en forme de plaque (146, 148) porte, sur un côté opposé à la surface d'élément d'outil respective (70, 72), une structure d'appui (150, 152),
la structure d'appui (150, 152) étant notamment en retrait dans la direction proximale par rapport à l'extrémité distale (126, 60) de l'élément d'outil (36, 38).

5. Instrument médical selon l'une des revendications précédentes,
**caractérisé en ce que**
a) la surface de préhension (116, 118, 120, 122) du ou des éléments de préhension est courbée de manière tangentielle continue
et/ou
b) la largeur (100, 102) présente au moins un maximum local (154, 156) entre les extrémités proximale et distale (124, 126, 58, 60) et que la surface de préhension (108, 110, 112, 114) s'étend dans la direction distale jusqu'au maximum local,
la largeur (100, 102) diminuant notamment de manière continue dans la direction distale à partir du maximum local (154, 156).

6. Instrument médical selon l'une des revendications précédentes,
**caractérisé en ce qu'**une distance entre le minimum local (128, 130) de la largeur (100, 102) et une extrémité proximale (132, 138) de la surface de préhension (116, 118, 120, 122) est supérieure à cette distance par rapport à l'extrémité distale (134, 140) de la surface de préhension (116, 118, 120, 122).

7. Instrument médical selon l'une des revendications précédentes,
**caractérisé en ce que**
a) le premier élément d'outil (36) et/ou le deuxième élément d'outil (38) sont réalisés de manière symétrique par rapport à un plan de symétrie (158) contenant un axe longitudinal (97, 66) de l'élément d'outil respectif (36, 38) définissant la direction d'extension des éléments d'outil (94, 96)
et/ou
b) une courbure de la surface de préhension (116, 118, 120, 122) d'au moins un élément de préhension (108, 110, 112, 114) définit un rayon de courbure compris dans une plage d'environ 5 mm à environ 30 mm, en particulier d'environ 15 mm,
et/ou
c) la surface de préhension (116, 118) du ou des éléments de préhension (108, 110) sur le premier élément d'outil (36) et la surface de préhension (120, 122) du ou des éléments de préhension (112, 114) sur le deuxième élément d'outil (38) sont agencées ou formées de manière à se chevaucher dans la direction d'extension des éléments d'outil (94, 96) ou décalées l'une par rapport à l'autre dans la direction distale ou proximale.

8. Instrument médical selon l'une des revendications précédentes, **caractérisé en ce que** le ou les éléments de préhension (108, 110, 112, 114)
a) sont formé ou conçus d'un seul tenant sur l'élément d'outil correspondant (36, 38)
ou
b) sont conçus pour pouvoir être reliés de manière amovible à l'élément d'outil correspondant (36, 38),
l'au moins un élément de préhension (108, 110, 112, 114) et l'élément d'outil correspondant (36, 38) étant notamment en prise par complémentarité de forme et/ou par force dans une position de liaison.

9. Instrument médical selon l'une des revendications précédentes,
**caractérisé en ce que**
a) l'au moins un élément de préhension (108, 110, 112, 114) est agencé ou formé plus près de l'extrémité distale (126, 66) que de l'extrémité proximale (124, 64) de l'élément d'outil correspondant (36, 38)
et/ou
b) la surface de préhension (116, 118, 120, 122) du ou des éléments de préhension (108, 110, 112, 114) s'étend transversalement, en particulier perpendiculairement, à la surface d'élément d'outil (70, 72) de l'élément d'outil respectif (36, 38)
et/ou
c) le ou les éléments de préhension (112, 114) présentent une autre surface de préhension (160, 162) et que l'autre surface de préhension est inclinée par rapport à la surface d'élément d'outil (72) de l'élément d'outil respectif (38)
et/ou
d) dans une position la plus rapprochée des au moins deux éléments d'outil (36, 38), une distance entre la première surface d'élément d'outil (70) et la deuxième surface d'élément d'outil (72) est constante ou sensiblement constante,
l'instrument (10) comprenant en particulier un dispositif d'écartement (76) pour prédéfinir une distance minimale (78) entre les au moins deux éléments d'outil (36, 38) dans la position la plus rapprochée,
le dispositif d'écartement (76) comprenant en outre en particulier au moins un élément d'écartement (74) qui est agencé ou formé en saillie par rapport à la première surface d'élément d'outil (70) ou à la deuxième surface d'élément d'outil (72),
le ou les éléments d'écartement (74) étant en outre en particulier réalisés en un matériau électriquement isolant, en particulier en céramique.

10. Instrument médical selon l'une des revendications précédentes, **caractérisé en ce que** les au moins deux éléments d'outil (36, 38)
a) sont réalisés dans un matériau électriquement conducteur, en particulier dans un conducteur métallique,
et/ou
b) sont réalisés sous forme de pièces moulées par injection de métal.

11. Instrument médical selon l'une des revendications précédentes,
**caractérisé en ce que** la première surface d'élément d'outil (70) et la deuxième surface d'élément d'outil (72) sont planes ou que la première surface d'élément d'outil (70) et la deuxième surface d'élément d'outil (72) sont courbées, en particulier d'une part concaves et d'autre part convexes de manière correspondante par rapport à la direction d'extension des éléments d'outil (94, 96).

12. Instrument médical selon l'une des revendications précédentes,
**caractérisé en ce que** le premier élément d'outil (36) et le deuxième élément d'outil (38) présentent chacun une fente (82, 84) s'étendant dans la direction d'extension des éléments d'outil (94, 96), qui traverse au moins en partie la première surface d'élément d'outil (70) et la deuxième surface d'élément d'outil (72),
la fente (82, 84) étant en particulier en forme de trou oblong.

13. Instrument médical selon l'une des revendications précédentes, **caractérisé en ce que** l'instrument
a) comprend un dispositif de coupe (86) avec un élément de coupe (88) qui est agencé de manière mobile ou conçu de manière mobile dans la direction d'extension des éléments d'outil (94, 96), le premier élément d'outil (36) et le deuxième élément d'outil (38) en particulier présentant chacun une fente (82, 84) s'étendant dans la direction d'extension des éléments d'outil (94, 96), laquelle fente traverse au moins par sections la première surface d'élément d'outil (70) et la deuxième surface d'élément d'outil (72), l'élément de coupe (88) présentant un tranchant (92) orienté dans la direction distale et le tranchant traversant au moins partiellement les fentes (82, 84) dans les éléments d'outil (36, 38) lorsque les éléments d'outil (36, 38) occupent la position maximale rapprochée,
et/ou
b) l'instrument (10) est réalisé sous la forme d'un instrument électrochirurgical (12) ou sous la forme d'un appareil d'agrafage et/ou
c) l'instrument comprend un dispositif d'actionnement (18) qui est agencé ou formé en coopération avec les éléments d'outil (36, 38) à une extrémité proximale (16) de l'instrument (10) pour déplacer ceux-ci les uns par rapport aux autres.

14. Instrument médical selon l'une des revendications précédentes,
**caractérisé en ce que**
a) la direction d'extension des éléments d'outil (94, 96) est rectiligne ou courbe
et/ou
b) les extrémités distales (126, 60) des deux éléments d'outil au moins (36, 38) sont arrondies.

15. Instrument médical selon l'une des revendications précédentes, **caractérisé en ce que** la surface de préhension (116, 118, 120, 122) du ou des éléments de préhension (108, 110, 112, 114)
a) présente une surface structurée, en particulier une surface structurée macroscopique
et/ou
b) est pourvue au moins partiellement, en particulier complètement, d'un revêtement,
le revêtement formant, en particulier,
- la surface structurée
et/ou
- est réalisé sous la forme d'un revêtement thermique, en particulier sous la forme d'un revêtement céramique.
